# EUROPEAN PATENT APPLICATION

(11) **EP 4 732 829 A1**
(43) Date of publication of application: **29.04.2026**
(21) Application number: 24825301.5
(22) Date of filing: 20.06.2024
(51) Int. Cl.: A61K 9/00, A61K 9/08, A61K 47/18, A61K 47/26, C07K 14/50, A61P 3/00

(54) **GLP-1-FC-FGF21 DOUBLE-TARGET FUSION PROTEIN COMPOSITION, AND INJECTION AND USE THEREOF**

(30) Priority: 20.06.2023 CN 202310741972
(71) Applicant: Sunshine Lake Pharma Co., Ltd., Dongguan, Guangdong 523000 (CN)
(72) Inventor: YU, Zhiyan, Dongguan, Guangdong 523871 (CN); HONG, Juan, Dongguan, Guangdong 523871 (CN); XIE, Can, Dongguan, Guangdong 523871 (CN); GONG, Qingwei, Dongguan, Guangdong 523871 (CN); LIU, Xiaoying, Dongguan, Guangdong 523871 (CN); XU, Jun, Dongguan, Guangdong 523871 (CN); YAN, Jiangyu, Dongguan, Guangdong 523871 (CN); GUO, Linfeng, Dongguan, Guangdong 523871 (CN); LI, Xiaoping, Dongguan, Guangdong 523871 (CN); LI, Wenjia, Dongguan, Guangdong 523871 (CN)
(74) Representative: Handley, Matthew Edward
(86) International application number: PCT/CN2024/100364
(87) International publication number: WO 2024/260414

(57) **Abstract**

A GLP-1-Fc-FGF21 double-target fusion protein composition and the use thereof. The composition comprises a GLP-1-Fc-FGF21 fusion protein and a buffer solution. In the composition, by means of adding the buffer solution, the generation of visible particles can be effectively controlled, and the stability of the GLP-1-Fc-FGF21 fusion protein is improved, so that the shelf life of a product can be prolonged. The composition can be used for treating and/or preventing metabolic diseases, such as diabetes, hyperlipidemia, obesity and diseases related to fatty liver.

## Description

### CROSS-REFERENCE TO RELATED APPLICATION

This application claims the priority and benefits of Chinese Patent Application No. 202310741972.5, filed with the State Intellectual Property Office of China on June 20, 2023, which is incorporated herein by reference in its entirety.

### FIELD OF THE INVENTION

The invention belongs to the biopharmaceutical technology field, and it specially relates to GLP-1-Fc-FGF21 dual-target fusion protein compositions, injections, and uses thereof. More specifically, it relates to a composition, injections, uses and injection device or container.

### BACKGROUND

Glucagon-like peptide-1 (GLP-1) is an incretin secreted by L cells in the small intestine, which stimulates the islet β cells to secrete insulin, thereby maintaining the insulin balance in the patient's body. GLP-1 exerts its effects indirectly through insulin and is only effective for type 2 diabetes, which limits its scope of application and efficacy. Additionally, there have been reports suggesting that GLP-1 may carry a potential risk of causing thyroid cancer.

FGF21 is a member of the fibroblast growth factor (FGF) family. It has the ability to facilitate the uptake of glucose by fat cells, thereby enhancing insulin sensitivity. Unlike insulin, FGF21 does not induce hypoglycemia or other related side effects. It is more effective in protecting beta islet cells and promoting their regeneration and repair. Moreover, due to its lack of mitogenic activity, it does not pose a potential risk of tumorigenesis. FGF21 holds promise as a therapeutic agent for type 1 diabetes. In addition, FGF21 also exhibits significant lipid-lowering effects, making it a potential candidate for lipid-lowering medication.

GLP-1-Fc-FGF21 dual-target fusion protein can be used to treat metabolic diseases such as diabetes, hyperlipidemia, obesity, and diseases associated with fatty liver. However, during storage, this dual-target fusion protein may undergo physical and chemical changes, such as denaturation, aggregation, precipitation, or fragmentation, which can affect its stability.

Therefore, there is an urgent need to develop a highly stable GLP-1-Fc-FGF21 dual-target fusion protein formulation.

### SUMMARY

The present invention aims to solve at least one of the technical problems existing in the prior art to some extent. To this end, the present invention provides a GLP-1-Fc-FGF21 dual-target fusion protein composition, which exhibits strong stability and can extend the shelf life of the product.

In one aspect of the present invention, a composition is proposed. According to an embodiment of the present invention, the composition includes: a GLP-1-Fc-FGF21 fusion protein and a buffer solution. In the composition of the present invention, the addition of the buffer solution can effectively control the generation of visible particles and aggregates, enhance the stability of the GLP-1-Fc-FGF21 fusion protein, and thereby extend the shelf life of the product.

According to an embodiment of the present invention, the GLP-1-Fc-FGF21 fusion protein comprises amino acid sequences as shown in SEQ ID NO: 1-12.

According to an embodiment of the present invention, the concentration of the GLP-1-Fc-FGF21 fusion protein in the composition is 1~100 mg/mL.

In some optional embodiments of the present invention, the concentration of the GLP-1-Fc-FGF21 fusion protein in the composition is 2~80 mg/mL.

In some optional embodiments of the present invention, the concentration of the GLP-1-Fc-FGF21 fusion protein in the composition is 2~70 mg/mL.

In some optional embodiments of the present invention, the concentration of the GLP-1-Fc-FGF21 fusion protein in the composition is 5~70 mg/mL.

In some optional embodiments of the present invention, the concentration of the GLP-1-Fc-FGF21 fusion protein in the composition is 2~65 mg/mL.

In some optional embodiments of the present invention, the concentration of the GLP-1-Fc-FGF21 fusion protein in the composition is 2~60 mg/mL.

In some optional embodiments of the present invention, the concentration of the GLP-1-Fc-FGF21 fusion protein in the composition is 2~55 mg/mL.

In some optional embodiments of the present invention, the concentration of the GLP-1-Fc-FGF21 fusion protein in the composition is 10~65 mg/mL.

In some optional embodiments of the present invention, the concentration of the GLP-1-Fc-FGF21 fusion protein in the composition is 20~60 mg/mL.

According to an embodiment of the present invention, the pH value of the composition is 6.2 ~8.0, preferably 6.4~8.0.

According to an embodiment of the present invention, the buffer solution includes at least one of a histidine buffer, a citrate buffer, a phosphate buffer, and a Tris buffer.

According to an embodiment of the present invention, the buffer solution includes a histidine buffer or a citrate buffer.

According to an embodiment of the present invention, the concentration of the buffer solution in the composition is 5~30 mM.

According to an embodiment of the present invention, the concentration of the buffer solution in the composition is 5~25 mM.

According to an embodiment of the present invention, the concentration of the buffer solution in the composition is 8~25 mM.

According to an embodiment of the present invention, the concentration of the buffer solution in the composition is 5~20 mM.

According to an embodiment of the present invention, the concentration of the buffer solution in the composition is 8 ~12 mM.

According to an embodiment of the present invention, the concentration of the buffer solution in the composition is 10~20 mM.

According to an embodiment of the present invention, the composition further includes at least one of an osmotic pressure regulator, a surfactant, a preservative, and a metal chelating agent.

According to an embodiment of the present invention, the osmotic pressure regulator includes at least one of mannitol, sucrose, sodium chloride, and trehalose.

According to an embodiment of the present invention, the concentration of the osmotic pressure regulator in the composition is 30~100 mg/mL or 1~10 mg/mL.

According to an embodiment of the present invention, the surfactant includes at least one of polysorbate 80, polysorbate 20, and poloxamer 188.

According to an embodiment of the present invention, the concentration of the surfactant in the composition is 0.1~10 mg/mL.

According to an embodiment of the present invention, the concentration of the surfactant in the composition is 0.1~1.5 mg/mL.

According to an embodiment of the present invention, the concentration of the surfactant in the composition is 6~10 mg/mL.

According to an embodiment of the present invention, the concentration of the surfactant in the composition is 0.1~0.7 mg/mL.

According to an embodiment of the present invention, the preservative is at least one of phenol and m-cresol.

According to an embodiment of the present invention, the concentration of the preservative in the composition is 0.1~10 mg/mL, preferably 0.1~5.0 mg/mL, and more preferably 1.0~5.0 mg/mL.

According to an embodiment of the present invention, the metal chelating agent is at least one of disodium edetate and calcium disodium edetate.

According to an embodiment of the present invention, the concentration of the metal chelating agent in the composition is 0.01~1 mg/mL, preferably 0.05~0.5 mg/mL.

According to an embodiment of the present invention, the buffer is a histidine buffer, and the concentration of the GLP-1-Fc-FGF21 fusion protein in the composition is 25~100 mg/mL, preferably 25~95 mg/mL, 25~90 mg/mL, or 25~85 mg/mL.

According to an embodiment of the present invention, the concentration of the GLP-1-Fc-FGF21 fusion protein in the composition is 25~80 mg/mL, and the buffer is a histidine buffer.

According to an embodiment of the present invention, the concentration of the GLP-1-Fc-FGF21 fusion protein in the composition is 25~55 mg/mL, and the buffer is a histidine buffer.

According to an embodiment of the present invention, the concentration of the histidine buffer in the composition is 5~30 mM.

According to an embodiment of the present invention, the concentration of the histidine buffer in the composition is 5~25 mM.

According to an embodiment of the present invention, the concentration of the histidine buffer in the composition is 8~25 mM.

According to an embodiment of the present invention, the concentration of the histidine buffer in the composition is 8~20 mM.

According to an embodiment of the present invention, the concentration of the histidine buffer in the composition is 10~20 mM.

According to an embodiment of the present invention, the concentration of the histidine buffer in the composition is 8~12 mM.

According to an embodiment of the present invention, the pH value of the composition is 6.7 ~ 7.5, preferably 6.9 ~ 7.5.

According to an embodiment of the present invention, the osmotic pressure regulator is mannitol, and the concentration of the osmotic pressure regulator in the composition is 30~50 mg/mL; or the osmotic pressure regulator is sucrose, and the concentration of the osmotic pressure regulator in the composition is 60~100 mg/mL, preferably 80~100 mg/mL; or the osmotic pressure regulator is trehalose, and the concentration of the osmotic pressure regulator in the composition is 40~100 mg/mL, preferably 40~70 mg/mL, and more preferably 50~70 mg/mL.

According to an embodiment of the present invention, the surfactant is polysorbate 80, and the concentration of the surfactant in the composition is 0.1~2 mg/mL, preferably 0.1~1.5 mg/mL, and more preferably 0.4~1.3 mg/mL; or the surfactant is polysorbate 20, and the concentration of the surfactant in the composition is 0.1~1.5 mg/mL, preferably 0.1~0.5 mg/mL.

According to an embodiment of the present invention, the concentration of the GLP-1-Fc-FGF21 fusion protein in the composition is 2~25 mg/mL, and the buffer is a citrate buffer.

According to an embodiment of the present invention, the concentration of the citrate buffer in the composition is 5~30 mM.

According to an embodiment of the present invention, the concentration of the citrate buffer in the composition is 5~25 mM.

According to an embodiment of the present invention, the concentration of the citrate buffer in the composition is 8~25 mM.

According to an embodiment of the present invention, the concentration of the citrate buffer in the composition is 8~20 mM.

According to an embodiment of the present invention, the concentration of the citrate buffer in the composition is 10~20 mM.

According to an embodiment of the present invention, the concentration of the citrate buffer in the composition is 8~12 mM.

According to an embodiment of the present invention, the pH value of the composition is 6.4 to 8.0.

According to an embodiment of the present invention, the osmotic pressure regulator is mannitol, and the concentration of the osmotic pressure regulator in the composition is 30~50 mg/mL; or the osmotic pressure regulator is sucrose, and the concentration of the osmotic pressure regulator in the composition is 60~100 mg/mL, preferably 80~100 mg/mL; or the osmotic pressure regulator is trehalose, and the concentration of the osmotic pressure regulator in the composition is 40~100 mg/mL, preferably 40~70 mg/mL, and more preferably 50~70 mg/mL.

According to an embodiment of the present invention, the osmotic pressure regulator is sodium chloride, and the concentration of the osmotic pressure regulator in the composition is 1~10 mg/mL, preferably 3~9 mg/mL.

According to an embodiment of the present invention, the surfactant includes at least one of polysorbate 80, polysorbate 20, and poloxamer 188, preferably polysorbate 80.

According to an embodiment of the present invention, the concentration of the surfactant in the composition is 0.1 to 10.0 mg/mL.

According to an embodiment of the present invention, the concentration of the surfactant in the composition is 0.1 to 1.5 mg/mL.

According to an embodiment of the present invention, the concentration of the surfactant in the composition is 0.1 to 1.0 mg/mL.

According to an embodiment of the present invention, the concentration of the surfactant in the composition is 6.0 to 10.0 mg/mL.

According to an embodiment of the present invention, the concentration of the surfactant in the composition is 0.1 to 0.5 mg/mL.

According to an embodiment of the present invention, the surfactant is poloxamer 188, and the concentration of the surfactant in the composition is 6~10 mg/mL; or the surfactant is polysorbate 80 or polysorbate 20, and the concentration of the surfactant in the composition is 0.1~1.5 mg/mL, preferably 0.1~1.0 mg/mL or 0.1~0.5 mg/mL.

According to an embodiment of the present invention, the dosage form of the composition is an injection.

In another aspect of the present invention, it provides an injection. According to an embodiment of the present invention, the injection comprises: a GLP-1-Fc-FGF21 fusion protein, a histidine buffer, mannitol, and optionally disodium edetate or calcium disodium edetate, as well as polysorbate 80 or polysorbate 20; wherein the GLP-1-Fc-FGF21 fusion protein has an amino acid sequence as shown in any one of SEQ ID NO: 1~12, and the concentration of the GLP-1-Fc-FGF21 fusion protein in the injection is 25~100 mg/mL; the concentration of the histidine buffer in the injection is 8~25 mM; the concentration of mannitol in the injection is 35~45 mg/mL; the concentration of polysorbate 80 in the injection is 0.4~1.3 mg/mL, or the concentration of polysorbate 20 in the injection is 0.1~0.5 mg/mL; the concentration of the metal chelating agent in the injection is 0.05~0.5 mg/mL; and the pH of the injection sis 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a histidine buffer, mannitol, and optionally disodium edetate or calcium disodium edetate, as well as polysorbate 80 or polysorbate 20; wherein the GLP-1-Fc-FGF21 fusion protein has an amino acid sequence as shown in any one of SEQ ID NO: 1~12, and the concentration of the GLP-1-Fc-FGF21 fusion protein in the injection is 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL; the concentration of the histidine buffer in the composition is 8~25 mM; the concentration of mannitol in the composition is 35~45 mg/mL; the concentration of polysorbate 80 in the composition is 0.4~1.3 mg/mL, or the concentration of polysorbate 20 in the composition is 0.1~0.5 mg/mL; the concentration of the metal chelating agent in the composition is 0.05~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a histidine buffer, mannitol, and optionally disodium edetate or calcium disodium edetate, as well as polysorbate 80 or polysorbate 20; wherein the GLP-1-Fc-FGF21 fusion protein has an amino acid sequence as shown in SEQ ID NO: 1, and the concentration of the GLP-1-Fc-FGF21 fusion protein in the injection is 25~50 mg/mL; the concentration of the histidine buffer in the injection is 10~20 mM; the concentration of mannitol in the injection is 35~45 mg/mL; the concentration of polysorbate 80 in the injection is 0.4~1.3 mg/mL, or the concentration of polysorbate 20 in the injection is 0.1~0.5 mg/mL; the concentration of the metal chelating agent in the injection is 0.05~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a histidine buffer, sucrose or trehalose, and optionally disodium edetate or calcium disodium edetate, as well as polysorbate 80 or polysorbate 20; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL; the concentration of the histidine buffer in the injection is 8~25 mM; the concentration of sucrose in the injection is 85~95 mg/mL, or the concentration of trehalose in the injection is 55~65 mg/mL; the concentration of polysorbate 80 in the injection is 0.4~1.3 mg/mL, or the concentration of polysorbate 20 in the injection is 0.1~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a histidine buffer, sucrose or trehalose, and optionally disodium edetate or calcium disodium edetate, as well as polysorbate 80 or polysorbate 20; wherein the GLP-1-Fc-FGF21 fusion protein has an amino acid sequence as shown in any one of SEQ ID NO: 1~12, and the concentration of the GLP-1-Fc-FGF21 fusion protein in the injection is 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL; the concentration of the histidine buffer in the composition is 8~20 mM; the concentration of sucrose in the composition is 85~95 mg/mL; the concentration of trehalose in the injection is 55~65 mg/mL; the concentration of polysorbate 80 in the composition is 0.4~1.3 mg/mL, or the concentration of polysorbate 20 in the composition is 0.1~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a histidine buffer, sucrose, and polysorbate 80 or polysorbate 20; wherein the GLP-1-Fc-FGF21 fusion protein comprises an amino acid sequence as shown in SEQ ID NO: 1, and the concentration of the GLP-1-Fc-FGF21 fusion protein in the injection is 25~50 mg/mL; the concentration of the histidine buffer in the injection is 10~20 mM; the concentration of sucrose in the injection is 85~95 mg/mL; the concentration of polysorbate 80 in the injection is 0.4~1.3 mg/mL, or the concentration of polysorbate 20 in the injection is 0.1~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a histidine buffer, trehalose, and polysorbate 80 or polysorbate 20; wherein the GLP-1-Fc-FGF21 fusion protein comprises an amino acid sequence as shown in SEQ ID NO: 1, and the concentration of the GLP-1-Fc-FGF21 fusion protein in the injection is 25~50 mg/mL; the concentration of the histidine buffer in the injection is 10~20 mM; the concentration of trehalose in the injection is 55~65 mg/mL; the concentration of polysorbate 80 in the injection is 0.4~1.3 mg/mL, or the concentration of polysorbate 20 in the injection is 0.1~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a citrate buffer, sucrose or trehalose, and optionally disodium edetate or calcium disodium edetate, as well as polysorbate 80 or polysorbate 20; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL; the concentration of the citrate buffer in the injection is 8~25 mM, preferably 10~20 mM; the concentration of sucrose in the injection is 85~95 mg/mL; the concentration of trehalose in the injection is 55~65 mg/mL; the concentration of polysorbate 80 in the injection is 0.4~1.3 mg/mL, or the concentration of polysorbate 20 in the injection is 0.1~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a citrate buffer, mannitol, and optionally disodium edetate or calcium disodium edetate, as well as polysorbate 80 or polysorbate 20; wherein the GLP-1-Fc-FGF21 fusion protein comprises an amino acid sequence as shown in any one of SEQ ID NO: 1~12, and the concentration of the GLP-1-Fc-FGF21 fusion protein in the injection solution is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL; the concentration of the citrate buffer in the injection is 8~25 mM, preferably 10~20 mM; the concentration of mannitol in the injection is 35~45 mg/mL; the concentration of polysorbate 80 in the injection is 0.4~1.5 mg/mL, or 0.4~1.3 mg/mL, or the concentration of polysorbate 20 in the injection is 0.1~0.5 mg/mL; the concentration of the metal chelating agent in the injection is 0.05~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate. In another aspect of the present invention, it provides an injection. According to an embodiment of the present invention, the injection comprises: a GLP-1-Fc-FGF21 fusion protein, a citrate buffer, mannitol, polysorbate 80, and optionally disodium edetate or calcium disodium edetate; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL; the concentration of the citrate buffer in the injection is 5~20 mM; the concentration of mannitol in the injection is 40~50 mg/mL; the concentration of polysorbate 80 in the injection is 0.1~1.5 mg/mL; the concentration of the metal chelating agent in the injection is 0.05~0.5 mg/mL; and the pH of the injection is 6.4~8.0. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a citrate buffer, mannitol, polysorbate 80, and optionally disodium edetate or calcium disodium edetate; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL; the concentration of the citrate buffer in the injection is 8~20 mM, 10~20 mM, or 10~15 mM; the concentration of mannitol in the injection is 40~50 mg/mL; the concentration of polysorbate 80 in the injection is 0.1~0.5 mg/mL; the concentration of the metal chelating agent in the injection is 0.05~0.5 mg/mL; and the pH of the injection is 6.4~8.0. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a citrate buffer, mannitol, polysorbate 80, and optionally disodium edetate or calcium disodium edetate; wherein the GLP-1-Fc-FGF21 fusion protein comprises an amino acid sequence as shown in SEQ ID NO: 1, and the concentration of the GLP-1-Fc-FGF21 fusion protein in the injection is 3~25 mg/mL; the concentration of the citrate buffer in the injection is 8~20 mM, 10~20 mM, or 10~15 mM; the concentration of mannitol in the injection is 40~50 mg/mL; the concentration of polysorbate 80 in the injection is 0.1~0.5 mg/mL; the concentration of the metal chelating agent in the injection is 0.05~0.5 mg/mL; and the pH of the injection is 6.4~8.0. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a citrate buffer, polysorbate 80, and optionally disodium edetate or calcium disodium edetate, as well as sucrose or trehalose; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL; the concentration of the citrate buffer in the injection is 5~20 mM; the concentration of sucrose in the injection is 80~95 mg/mL; the concentration of trehalose in the injection is 55~65 mg/mL; the concentration of polysorbate 80 in the injection is 0.1~1.5 mg/mL; and the pH of the injection is 6.4~8.0. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In another aspect of the present invention, it provides an injection. According to an embodiment of the present invention, the injection comprises: a GLP-1-Fc-FGF21 fusion protein, a citrate buffer, polysorbate 80, as well as sucrose or trehalose, and optionally disodium edetate or calcium disodium edetate; wherein the GLP-1-Fc-FGF21 fusion protein comprises an amino acid sequence as shown in SEQ ID NO: 1, and the concentration of the GLP-1-Fc-FGF21 fusion protein in the injection is 3~25 mg/mL; the concentration of the citrate buffer in the injection is 8~20 mM, 10~20 mM, or 10~15 mM; the concentration of sucrose in the injection is 80~95 mg/mL; the concentration of trehalose in the injection is 55~65 mg/mL; the concentration of polysorbate 80 in the injection is 0.1~0.5 mg/mL; and the pH of the injection is 6.4~8.0. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present invention, the concentration of phenol in the composition or injection is 0~7 mg/mL, preferably 3~7 mg/mL.

In an optional embodiment of the present invention, the concentration of m-cresol in the composition or injection is 0~4 mg/mL, preferably 2~4 mg/mL.

In another aspect of the invention, it provides a use of the aforementioned composition or injection in the preparation of a medicament for treating and/or preventing metabolic diseases.

According to an embodiment of the present invention, the metabolic diseases include diabetes, hyperlipidemia, obesity, and diseases associated with fatty liver.

According to an embodiment of the present invention, the diseases associated with fatty liver include non-alcoholic fatty liver disease (NAFLD), non-alcoholic steatohepatitis (NASH), liver fibrosis, and cirrhosis.

In another aspect of the invention, it provides an injection device or container. According to an embodiment of the present invention, the injection device or container comprises: the aforementioned composition or the aforementioned injection.

According to an embodiment of the present invention, the injection device or container comprises at least one selected from a vial and a syringe.

According to an embodiment of the present invention, the syringe comprises at least one selected from an auto-injector and a pre-filled syringe.

Additional aspects and advantages of the invention will be partially set forth in the following description, and will partially become apparent from the following description, or may be learned through the practice of the invention.

### Description of the drawings

The above and/or additional aspects and advantages of the present invention will become apparent and readily understood from the description of the embodiments in conjunction with the accompanying drawings, wherein:
Figure 1 shows the high-temperature SEC (Size Exclusion Chromatography) aggregate growth trend chart of different formulations in step 7 of Example 1 of the invention;
Figure 2 shows the high-temperature RP-HPLC (Reverse Phase High-Performance Liquid Chromatography) purity (total related substances) growth trend chart of different formulations in step 7 of Example 1 of the invention.

### EXAMPLES

The embodiments of the present invention are described in detail below. The embodiments described below are exemplary and are intended solely to explain the invention, and should not be construed as limiting the invention.

It should be noted that the terms "first" and "second" are used for descriptive purposes only and should not be interpreted as indicating or implying relative importance or implicitly specifying the quantity of the indicated technical features. Thus, features defined as "first" or "second" may explicitly or implicitly include one or more of such features. Furthermore, in the description of the present invention, unless otherwise specified, the term "a plurality of" means two or more.

The endpoints and any values of the ranges disclosed herein are not limited to the precise ranges or values, and these ranges or values should be understood to include values close to these ranges or values. For numerical ranges, the endpoint values of each range, the endpoint values of each range and individual point values, as well as individual point values, may be combined with each other to obtain one or more new numerical ranges, which should be considered as specifically disclosed herein.

In this document, the term "comprise" or "include" is an open-ended expression, meaning that it includes the content specified by the present invention but does not exclude other contents.

In this document, the terms "optionally," "optional," or "option" generally mean that the subsequently described event or condition may but does not necessarily occur, and the description includes situations where the event or condition occurs and situations where it does not occur.

In this document, the term "treatment" refers to achieving the desired pharmacological and/or physiological effects. The effects may be preventive in terms of completely or partially preventing a disease or its symptoms, and/or therapeutic in terms of partially or completely curing the disease and/or adverse effects caused by the disease. As used herein, "treatment" encompasses diseases in mammals, particularly humans, and includes: (a) preventing the occurrence of a disease or condition in an individual who is susceptible to but has not yet been diagnosed with the disease; (b) inhibiting the disease, such as halting its progression; or (c) alleviating the disease, such as reducing symptoms associated with the disease. As used herein, "treatment" covers any administration of a composition, injection, or drug to an individual to treat, cure, alleviate, improve, relieve, or inhibit the individual's disease, including but not limited to administering a drug containing the composition or injection solution described herein to an individual in need.

The present invention proposes a composition, an injection, their uses, and an injection device or container, which will be described in detail below.

### Composition

In one aspect of the present invention, a composition is proposed. According to an embodiment of the present invention, the composition includes: a GLP-1-Fc-FGF21 fusion protein and a buffer solution. In the composition of the present invention, the addition of the buffer solution can effectively control the generation of visible particles and aggregates, enhance the stability of the GLP-1-Fc-FGF21 fusion protein, and thereby extend the shelf life of the product.

In some optional embodiments of the present invention, the GLP-1-Fc-FGF21 fusion protein comprises an amino acid sequence as shown in SEQ ID NO: 1~12.

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGASQGLSPSYAS（SEQ ID
NO:1）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGGSQGLSPSYAS（SEQ ID
NO:2）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY

SKLTVDKSRWQEGNVFSCSVMHEALHNHYTQKSLSLSLGGGGGSGGGGSGGGGSHPIPD
SSPLLQFGGQVRQVYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPAVFLPLPGLPPALPEPPGILAPQPPDVGSSDPLHMVGGSQGLSPSYAS（SEQ ID
NO:3）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGGSQGLSPSYES（SEQ ID
NO:4）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTECHLEIREDGTVGCAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGASQGLSPSYAS（SEQ ID
NO:5）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTECHLEIREDGTVGCAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGGSQGLSPSYAS（SEQ ID

NO:6)；

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGASQGLSPSYES（SEQ ID
NO:7）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQRYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGASQGLSPSYAS（SEQ ID
NO:8）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQRYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGGSQGLSPSYAS（SEQ ID
NO:9）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ

VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQQYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPAQFLPLPGLPPALPEPPGILAPQPPDVGSSDPLHMVGGSQGLSPSYAS（SEQ ID
NO:10）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQQYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPAQFLPLPGLPPALPEPPGILAPQPPDVGSSDPLHMVGASQGLSPSYAS（SEQ ID
NO:11）;

HGEGTFTSDVSSYLEEQAAKEFIAWLVKGGGGGGGSGGGGSGGGGSESKYGPPCPP
CPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SKLTVDKSRWQEGNVFSCSVMHEALHNHYTQKSLSLSLGGGGGSGGGGSGGGGSHPIPD
SSPLLQFGGQVRQVYLYTDDAQQTEAHLEIREDGTVGGAADQSPESLLQLKALKPGVIQI
LGVKTSRFLCQRPDGALYGSLHFDPEACSFRERLLEDGYNVYQSEAHGLPLHLPGNKSPH
RDPAPRGPARFLPLPGLPPALPEPPGILAPQPPDVGSSDPLHMVGASQGLSPSYES（SEQ ID
NO:12）.

In some optional embodiments of the present invention, the GLP-1-Fc-FGF21 fusion protein comprises an amino acid sequence as shown in SEQ ID NO: 1~12.

In some optional embodiments of the present invention, the GLP-1-Fc-FGF21 fusion protein comprises an amino acid sequence as shown in SEQ ID NO: 1.

In some optional embodiments of the present invention, the GLP-1-Fc-FGF21 fusion protein comprises an amino acid sequence as shown in SEQ ID NO: 2.

In some optional embodiments of the present invention, the GLP-1-Fc-FGF21 fusion protein comprises an amino acid sequence as shown in SEQ ID NO: 3.

In some optional embodiments of the present invention, the GLP-1-Fc-FGF21 fusion protein comprises an amino acid sequence as shown in SEQ ID NO: 4.

In some optional embodiments of the present invention, the GLP-1-Fc-FGF21 fusion protein comprises an amino acid sequence as shown in SEQ ID NO: 5.

In some optional embodiments of the present invention, the GLP-1-Fc-FGF21 fusion protein comprises an amino acid sequence as shown in SEQ ID NO: 6.

In some optional embodiments of the present invention, the GLP-1-Fc-FGF21 fusion protein comprises an amino acid sequence as shown in SEQ ID NO: 7.

In some optional embodiments of the present invention, the GLP-1-Fc-FGF21 fusion protein comprises an amino acid sequence as shown in SEQ ID NO: 8.

In some optional embodiments of the present invention, the GLP-1-Fc-FGF21 fusion protein comprises an amino acid sequence as shown in SEQ ID NO: 9.

In some optional embodiments of the present invention, the GLP-1-Fc-FGF21 fusion protein comprises an amino acid sequence as shown in SEQ ID NO: 10.

In some optional embodiments of the present invention, the GLP-1-Fc-FGF21 fusion protein comprises an amino acid sequence as shown in SEQ ID NO: 11.

In some optional embodiments of the present invention, the GLP-1-Fc-FGF21 fusion protein comprises an amino acid sequence as shown in SEQ ID NO: 12.

In some optional embodiments of the present invention, the concentration of the GLP-1-Fc-FGF21 fusion protein in the composition is 1~100 mg/mL, such as 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 100 mg/mL, or any range value between two of these values as endpoints, such as 2~99 mg/mL, 2~95 mg/mL, 2~90 mg/mL, 2~85 mg/mL, 2~80 mg/mL, 2~75 mg/mL, 2~70 mg/mL, 2~65 mg/mL, 2~60 mg/mL, 2~55 mg/mL, 5~99 mg/mL, 5~95 mg/mL, 5~90 mg/mL, 5~85 mg/mL, 5~80 mg/mL, 5~75 mg/mL, 5~70 mg/mL, 5~65 mg/mL, 5~60 mg/mL, 5~55 mg/mL, 10~99 mg/mL, 10~95 mg/mL, 10~90 mg/mL, 10~85 mg/mL, 10~80 mg/mL, 10~75 mg/mL, 10~70 mg/mL, 10~65 mg/mL, 10~60 mg/mL, 10~55 mg/mL, 25~99 mg/mL, 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, 30~99 mg/mL, 30~95 mg/mL, 30~90 mg/mL, 30~85 mg/mL, 30~80 mg/mL, 30~75 mg/mL, 30~70 mg/mL, 30~65 mg/mL, 30~60 mg/mL, 30~55 mg/mL, 35~99 mg/mL, 35~95 mg/mL, 35~90 mg/mL, 35~85 mg/mL, 35~80 mg/mL, 35~75 mg/mL, 35~70 mg/mL, 35~65 mg/mL, 35~60 mg/mL, 35~55 mg/mL, 40~99 mg/mL, 40~95 mg/mL, 40~90 mg/mL, 40~85 mg/mL, 40~80 mg/mL, 40~75 mg/mL, 40~70 mg/mL, 40~65 mg/mL, 40~60 mg/mL, 40~55 mg/mL.

In some optional embodiments of the present invention, the concentration of the GLP-1-Fc-FGF21 fusion protein in the composition is 2~55 mg/mL, such as 2~25 mg/mL, 3~25 mg/mL, 10~25 mg/mL, 10~20 mg/mL, 15~25 mg/mL, 15~20 mg/mL, 25~100 mg/mL, 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, 25~50 mg/mL, 25~40 mg/mL, 40~55 mg/mL, 40~50 mg/mL, 45~55 mg/mL, or 45~50 mg/mL.

In some optional embodiments of the present invention, the pH value of the composition is 6.4~8.0, such as 6.4, 6.5, 6.6, 6.7, 6.8, 6.9, 7.0, 7.1, 7.2, 7.3, 7.4, 7.5, 7.6, 7.7, 7.8, 7.9, or 8.0, or any range value between two of these values as endpoints. This can further enhance the stability of the fusion protein, for example, the pH value of the composition is 6.2~8.0 or 6.9~7.5.

In some optional embodiments of the present invention, the buffer solution includes at least one of a histidine buffer, a citrate buffer, a phosphate buffer, and a Tris buffer.

In this document, unless otherwise specified, the "citrate buffer" or "citrate buffer solution" may be selected from citric acid, sodium citrate, or a mixture of citric acid and sodium citrate, all of which fall within the scope of protection of this application.

In some optional embodiments of the present invention, the phosphate buffer is selected from a combination of disodium hydrogen phosphate and sodium dihydrogen phosphate or a combination of dipotassium hydrogen phosphate and potassium dihydrogen phosphate.

In some optional embodiments of the present invention, the histidine buffer includes histidine and/or histidine hydrochloride.

In some optional embodiments of the present invention, the histidine buffer includes histidine and histidine hydrochloride.

In some optional embodiments of the present invention, the histidine buffer includes histidine or histidine hydrochloride.

In some optional embodiments of the present invention, the citrate buffer includes citric acid and/or sodium citrate.

In some optional embodiments of the present invention, the citrate buffer includes citric acid and sodium citrate.

In some optional embodiments of the present invention, the citrate buffer includes citric acid or sodium citrate.

In some optional embodiments of the present invention, the Tris buffer includes Tris and/or Tris-HCl.

In some optional embodiments of the present invention, the Tris buffer includes Tris and Tris-HCl.

In some optional embodiments of the present invention, the Tris buffer includes Tris or Tris-HCl.

In some optional embodiments of the present invention, the buffer includes a histidine buffer or a citrate buffer.

In some optional embodiments of the present invention, the concentration of the buffer in the composition is 5~30 mM, such as 5 mM, 6 mM, 7 mM, 8 mM, 9 mM, 10 mM, 11 mM, 11.29 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, or 30 mM, or any range value between two of these values as endpoints (e.g., 5~25 mM, 8~25 mM, 8~12 mM, 10~20 mM).

In some optional embodiments of the present invention, the composition further includes at least one of an osmotic pressure regulator, a surfactant, a preservative, and a metal chelating agent. This can further enhance the stability of the fusion protein.

In some optional embodiments of the present invention, the osmotic pressure regulator includes at least one of mannitol, sucrose, sodium chloride, and trehalose.

In some optional embodiments of the present invention, the concentration of the osmotic pressure regulator in the composition is 30~100 mg/mL, such as 30 mg/mL, 35 mg/mL, 40 mg/mL, 41 mg/mL, 41.4 mg/mL, 41.5 mg/mL, 42 mg/mL, 42.4 mg/mL, 42.5 mg/mL, 43 mg/mL, 43.4 mg/mL, 43.5 mg/mL, 44 mg/mL, 44.4 mg/mL, 44.5 mg/mL, 45 mg/mL, 45.4 mg/mL, 45.5 mg/mL, 46 mg/mL, 46.4 mg/mL, 46.5 mg/mL, 47 mg/mL, 47.4 mg/mL, 47.5 mg/mL, 48 mg/mL, 48.4 mg/mL, 48.5 mg/mL, 49 mg/mL, 49.4 mg/mL, 49.5 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, or 100 mg/mL, or any range value between two of these values as endpoints.

In some optional embodiments of the present invention, the concentration of the osmotic pressure regulator in the composition is 1~10 mg/mL, such as 1 mg/mL, 2 mg/mL, 3 mg/mL, 4 mg/mL, 5 mg/mL, 6 mg/mL, 7 mg/mL, 8 mg/mL, 9 mg/mL, or 10 mg/mL, or any range value between two of these values as endpoints.

In some optional embodiments of the present invention, the surfactant includes at least one of polysorbate 80, polysorbate 20, and poloxamer 188.

In some optional embodiments of the present invention, the concentration of the surfactant in the composition is 0.1~10 mg/mL, such as 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, 2.0 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, or 10 mg/mL, or any range value between two of these values as endpoints (e.g., 1~1.5 mg/mL, 6~10 mg/mL, 0.1~0.7 mg/mL).

For example, the concentration of the surfactant in the composition is 0.1~1.5 mg/mL or 0.1~1 mg/mL.

In some optional embodiments of the present invention, the preservative includes at least one of phenol and m-cresol.

According to embodiments of the present invention, the concentration of the preservative in the composition is 0.1~10 mg/mL, such as 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, 9.0 mg/mL, 9.5 mg/mL, or 10.0 mg/mL, such as 1.0~5.0 mg/mL.

In some optional embodiments of the present invention, the preservative is phenol.

According to embodiments of the present invention, the concentration of phenol in the composition is 1.0~5.0 mg/mL, such as 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, 9.0 mg/mL, 9.5 mg/mL, or 10.0 mg/mL, e.g., 2~7 mg/mL or 3.0~4.0 mg/mL.

In some optional embodiments of the present invention, the preservative is m-cresol.

According to embodiments of the present invention, the concentration of m-cresol in the composition is 1.0~5.0 mg/mL, such as 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, 9.0 mg/mL, 9.5 mg/mL, or 10.0 mg/mL, e.g., 3.0~4.0 mg/mL.

In some optional embodiments of the present invention, the preservative includes both phenol and m-cresol.

According to embodiments of the present invention, the concentration of phenol in the composition is 1.0~5.0 mg/mL (such as 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, 9.0 mg/mL, 9.5 mg/mL, or 10.0 mg/mL), and the concentration of m-cresol in the composition is 0.1~1.0 mg/mL (such as 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL).

In some optional embodiments of the present invention, the metal chelating agent includes at least one of disodium edetate (EDTA-2Na) and calcium disodium edetate (EDTA-CaNa₂).

In some optional embodiments of the present invention, the metal chelating agent is disodium edetate (EDTA-2Na).

In some optional embodiments of the present invention, the metal chelating agent is calcium disodium edetate (EDTA-CaNa₂).

In some optional embodiments of the present invention, the concentration of the metal chelating agent in the composition is 0.01~1 mg/mL, such as 0.01 mg/mL, 0.02 mg/mL, 0.03 mg/mL, 0.04 mg/mL, 0.05 mg/mL, 0.06 mg/mL, 0.07 mg/mL, 0.08 mg/mL, 0.09 mg/mL, 0.1 mg/mL, 0.11 mg/mL, 0.115 mg/mL, 0.12 mg/mL, 0.13 mg/mL, 0.14 mg/mL, 0.15 mg/mL, 0.16 mg/mL, 0.17 mg/mL, 0.18 mg/mL, 0.19 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.65 mg/mL, 0.7 mg/mL, 0.75 mg/mL, 0.8 mg/mL, 0.85 mg/mL, 0.9 mg/mL, 0.95 mg/mL, or 1.0 mg/mL, or any range value between two of these values as endpoints (e.g., 0.05~0.5 mg/mL, 0.08~0.12 mg/mL, 0.1~0.12 mg/mL).

Exemplarily, the concentration of the metal chelating agent in the composition is 0.08~0.12 mg/mL. In the embodiments of the present invention, whether in a citrate system or a histidine buffer system, the stability of the fusion protein formulation remains good regardless of whether EDTA sodium salt is added. Thus, both the inclusion or exclusion of EDTA sodium salt falls within the scope of protection of the present invention.

In some optional embodiments of the present invention, the buffer is a histidine buffer, and the concentration of the GLP-1-Fc-FGF21 fusion protein in the composition is 25~100 mg/mL, such as 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, or 100 mg/mL, or any range value between two of these values as endpoints (e.g.,25~75 mg/mL, 25~70 mg/mL, 25~65 mg/mL, 25~60 mg/mL, 25~55 mg/mL, 25~50 mg/mL, 25~40 mg/mL, 40~80 mg/mL, 40~75 mg/mL, 40~70 mg/mL, 40~65 mg/mL, 40~60 mg/mL, 40~55 mg/mL, 40~50 mg/mL, 45~80 mg/mL, 45~75 mg/mL, 45~70 mg/mL, 45~65 mg/mL, 45~60 mg/mL, 45~55 mg/mL, or 45~50 mg/mL).

In some optional embodiments of the present invention, the concentration of the GLP-1-Fc-FGF21 fusion protein in the composition is 25~55 mg/mL, and the buffer is a histidine buffer. The inventors unexpectedly discovered that when the final concentration of the fusion protein is 25~55 mg/mL, the addition of the histidine buffer can effectively control the generation of visible particles in the composition. This not only improves the physical stability of the fusion protein and reduces potential safety risks but also effectively controls the rate of chemical changes, maintaining stability under high-temperature, accelerated, long-term, freeze-thaw, shaking, and simulated transportation conditions. The resulting composition has an extended shelf life.

In some optional embodiments of the present invention, the concentration of the histidine buffer in the composition is 5~30 mM, such as 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, or 30 mM, or any range value between two of these values as endpoints (e.g., 5~25 mM, 8~25 mM, 8~20 mM, 10~20 mM, or 8~12 mM).

In some optional embodiments of the present invention, the concentration of the histidine buffer in the composition is 10~20 mM.

In some optional embodiments of the present invention, the pH value of the composition is 6.7~7.5, preferably 6.9~7.5.

In some optional embodiments of the present invention, the osmotic pressure regulator is mannitol, and its concentration in the composition is 30~50 mg/mL; or the osmotic pressure regulator is sucrose, and its concentration in the composition is 60~100 mg/mL, preferably 80~100 mg/mL; or the osmotic pressure regulator is trehalose, and its concentration in the composition is 40~100 mg/mL, preferably 40~70 mg/mL, 50~70 mg/mL, or 55~65 mg/mL; or the osmotic pressure regulator is sodium chloride, and its concentration in the composition is 1~10 mg/mL, preferably 3~9 mg/mL.

In some optional embodiments of the present invention, the surfactant is polysorbate 80, and its concentration in the composition is 0.1~2 mg/mL, such as 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, or 2.0 mg/mL, or any range value between two of these values as endpoints (e.g., 0.1~1.5 mg/mL, 0.2~2 mg/mL, or 0.4~1.3 mg/mL); or

in some optional embodiments of the present invention, the surfactant is polysorbate 20, and its concentration in the composition is 0.1~1.5 mg/mL, such as 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, or 1.5 mg/mL, or any range value between two of these values as endpoints (e.g., 0.1~0.5 mg/mL).

In some optional embodiments of the present invention, the concentration of the GLP-1-Fc-FGF21 fusion protein in the composition is 2~25 mg/mL, and the buffer is a citrate buffer. The inventors unexpectedly discovered that when the final concentration of the fusion protein is 2~25 mg/mL, the addition of the citrate buffer can effectively control the generation of visible particles in the composition. This improves the physical and chemical stability of the fusion protein, reduces potential safety risks, and extends the shelf life of the composition.

In some optional embodiments of the present invention, the concentration of the citrate buffer in the composition is 5~30 mM, such as 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, or 30 mM, or any range value between two of these values as endpoints (e.g., 5~25 mM, 8~25 mM, 8~20 mM, 10~20 mM, 8~12 mM, 5~15 mM, or 10~15 mM).

In some optional embodiments of the present invention, the pH value of the composition is 6.4~8.0.

In some optional embodiments of the present invention, the osmotic pressure regulator is mannitol, and its concentration in the composition is 30~50 mg/mL; or the osmotic pressure regulator is sucrose, and its concentration in the composition is 60~100 mg/mL, preferably 80~100 mg/mL; or the osmotic pressure regulator is trehalose, and its concentration in the composition is 40~100 mg/mL, preferably 40~70 mg/mL, more preferably 50~70 mg/mL.

According to an embodiment of the present invention, the osmotic pressure regulator is sodium chloride, and the concentration of the osmotic pressure regulator in the composition is 1~10 mg/mL, preferably 3~9 mg/mL.

In some optional embodiments of the present invention, the surfactant includes at least one of polysorbate 80, polysorbate 20, and poloxamer 188, preferably polysorbate 80.

In some optional embodiments of the present invention, the concentration of the surfactant in the composition is 0.1~10.0 mg/mL, such as 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 2.0 mg/mL, 3.0 mg/mL, 4.0 mg/mL, 5.0 mg/mL, 6.0 mg/mL, 7.0 mg/mL, 8.0 mg/mL, 9.0 mg/mL, or 10.0 mg/mL, or any range value between two of these values as endpoints (e.g., 0.1~1.5 mg/mL, 0.1~1.0 mg/mL, 6.0~10.0 mg/mL, 0.1~0.5 mg/mL, or 0.1~0.3 mg/mL).

In some optional embodiments of the present invention, the surfactant is poloxamer 188, and its concentration in the composition is 6~10 mg/mL; or the surfactant is polysorbate 80 or polysorbate 20, and its concentration in the composition is 0.1~1.5 mg/mL, preferably 0.1~1 mg/mL or 0.1~0.5 mg/mL.

In some optional embodiments of the present invention, the dosage form of the composition is an injectable formulation.

### Injection

In another aspect of the present invention, it provides an injection. According to an embodiment of the present invention, the injection comprises: a GLP-1-Fc-FGF21 fusion protein, a histidine buffer, mannitol, optionally disodium edetate or calcium disodium edetate, and polysorbate 80 or polysorbate 20; wherein the GLP-1-Fc-FGF21 fusion protein has an amino acid sequence as shown in any one of SEQ ID NOs: 1-12, and the concentration of the GLP-1-Fc-FGF21 fusion protein in the injection is 25-100 mg/mL; the concentration of the histidine buffer in the composition is 8-25 mM; the concentration of mannitol in the composition is 35-45 mg/mL; the concentration of polysorbate 80 in the composition is 0.4-1.3 mg/mL, or the concentration of polysorbate 20 in the composition is 0.1-0.5 mg/mL; the concentration of the metal chelating agent in the composition is 0.05-0.5 mg/mL; the pH of the injection is 6.9-7.5. The injection of the present invention can effectively control the generation of visible particles and aggregates, improve the physical and chemical stability of the fusion protein, thereby reducing potential safety risks and extending the shelf life.

According to an embodiment of the present invention, the concentration of the GLP-1-Fc-FGF21 fusion protein in the injection is 25-95 mg/mL.

According to an embodiment of the present invention, the concentration of the GLP-1-Fc-FGF21 fusion protein in the injection is 25-90 mg/mL.

According to an embodiment of the present invention, the concentration of the GLP-1-Fc-FGF21 fusion protein in the injection is 25-85 mg/mL.

According to an embodiment of the present invention, the concentration of the GLP-1-Fc-FGF21 fusion protein in the injection is 25-80 mg/mL.

According to an embodiment of the present invention, the concentration of the GLP-1-Fc-FGF21 fusion protein in the injection is 25-75 mg/mL.

According to an embodiment of the present invention, the concentration of the GLP-1-Fc-FGF21 fusion protein in the injection is 25-70 mg/mL.

According to an embodiment of the present invention, the concentration of the GLP-1-Fc-FGF21 fusion protein in the injection is 25-65 mg/mL.

According to an embodiment of the present invention, the concentration of the GLP-1-Fc-FGF21 fusion protein in the injection is 25-60 mg/mL.

According to an embodiment of the present invention, the concentration of the GLP-1-Fc-FGF21 fusion protein in the injection is 25-55 mg/mL.

According to an embodiment of the present invention, the concentration of the GLP-1-Fc-FGF21 fusion protein in the injection is 25-50 mg/mL.

In some optional embodiments of the present invention, the GLP-1-Fc-FGF21 fusion protein comprises an amino acid sequence as shown in SEQ ID NO: 1. In some optional embodiments of the present invention, the GLP-1-Fc-FGF21 fusion protein comprises an amino acid sequence as shown in SEQ ID NO: 2. In some optional embodiments of the present invention, the GLP-1-Fc-FGF21 fusion protein comprises an amino acid sequence as shown in SEQ ID NO: 3. In some optional embodiments of the present invention, the GLP-1-Fc-FGF21 fusion protein comprises an amino acid sequence as shown in SEQ ID NO: 4. In some optional embodiments of the present invention, the GLP-1-Fc-FGF21 fusion protein comprises an amino acid sequence as shown in SEQ ID NO: 5. In some optional embodiments of the present invention, the GLP-1-Fc-FGF21 fusion protein comprises an amino acid sequence as shown in SEQ ID NO: 6. In some optional embodiments of the present invention, the GLP-1-Fc-FGF21 fusion protein comprises an amino acid sequence as shown in SEQ ID NO: 7. In some optional embodiments of the present invention, the GLP-1-Fc-FGF21 fusion protein comprises an amino acid sequence as shown in SEQ ID NO: 8. In some optional embodiments of the present invention, the GLP-1-Fc-FGF21 fusion protein comprises an amino acid sequence as shown in SEQ ID NO: 9. In some optional embodiments of the present invention, the GLP-1-Fc-FGF21 fusion protein comprises an amino acid sequence as shown in SEQ ID NO: 10. In some optional embodiments of the present invention, the GLP-1-Fc-FGF21 fusion protein comprises an amino acid sequence as shown in SEQ ID NO: 11. In some optional embodiments of the present invention, the GLP-1-Fc-FGF21 fusion protein comprises an amino acid sequence as shown in SEQ ID NO: 12.

According to an embodiment of the present invention, the injection comprises: a GLP-1-Fc-FGF21 fusion protein, a histidine buffer, mannitol, optionally disodium edetate or calcium disodium edetate, and polysorbate 80 or polysorbate 20; wherein the GLP-1-Fc-FGF21 fusion protein has an amino acid sequence as shown in SEQ ID NO: 1, and the concentration of the GLP-1-Fc-FGF21 fusion protein in the injection is 25-50 mg/mL; the concentration of the histidine buffer in the composition is 10-20 mM; the concentration of mannitol in the composition is 35-45 mg/mL; the concentration of polysorbate 80 in the composition is 0.4-1.3 mg/mL, or the concentration of polysorbate 20 in the composition is 0.1-0.5 mg/mL; the concentration of the metal chelating agent in the composition is 0.05-0.5 mg/mL; the pH of the injection is 6.9-7.5.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present application, the concentration of phenol in the composition or injection is 0-7 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, or any range between two of these values as endpoints (e.g., 3-7 mg/mL, 2-4 mg/mL).

In another optional embodiment of the present application, the concentration of m-cresol in the composition or injection is 0-4 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, or any range between two of these values as endpoints (e.g., 2-4 mg/mL).

In another aspect of the present invention, it provides an injection. According to an embodiment of the present invention, the injection comprises: a GLP-1-Fc-FGF21 fusion protein, a histidine buffer, sucrose or trehalose, optionally disodium edetate or calcium disodium edetate, and polysorbate 80 or polysorbate 20; wherein the GLP-1-Fc-FGF21 fusion protein has an amino acid sequence as shown in any one of SEQ ID NOs: 1-12, and the concentration of the GLP-1-Fc-FGF21 fusion protein in the injection is 25-100 mg/mL; the concentration of the histidine buffer in the composition is 8-20 mM; the concentration of sucrose in the composition is 85-95 mg/mL; the concentration of trehalose in the injection is 55-65 mg/mL; the concentration of polysorbate 80 in the composition is 0.4-1.3 mg/mL, or the concentration of polysorbate 20 in the composition is 0.1-0.5 mg/mL; the pH of the injection is 6.9-7.5. The injection of the present invention can effectively control the generation of visible particles and aggregates, improve the physical and chemical stability of the fusion protein, thereby reducing potential safety risks and extending the shelf life.

According to an embodiment of the present invention, the concentration of the GLP-1-Fc-FGF21 fusion protein in the injection is 25-95 mg/mL.

According to an embodiment of the present invention, the concentration of the GLP-1-Fc-FGF21 fusion protein in the injection is 25-90 mg/mL.

According to an embodiment of the present invention, the concentration of the GLP-1-Fc-FGF21 fusion protein in the injection is 25-85 mg/mL.

According to an embodiment of the present invention, the concentration of the GLP-1-Fc-FGF21 fusion protein in the injection is 25-80 mg/mL.

According to an embodiment of the present invention, the concentration of the GLP-1-Fc-FGF21 fusion protein in the injection is 25-75 mg/mL.

According to an embodiment of the present invention, the concentration of the GLP-1-Fc-FGF21 fusion protein in the injection is 25-70 mg/mL.

According to an embodiment of the present invention, the concentration of the GLP-1-Fc-FGF21 fusion protein in the injection is 25-65 mg/mL.

According to an embodiment of the present invention, the concentration of the GLP-1-Fc-FGF21 fusion protein in the injection is 25-60 mg/mL.

According to an embodiment of the present invention, the concentration of the GLP-1-Fc-FGF21 fusion protein in the injection is 25-55 mg/mL.

According to an embodiment of the present invention, the concentration of the GLP-1-Fc-FGF21 fusion protein in the injection is 25-50 mg/mL.

In some optional embodiments of the present invention, the GLP-1-Fc-FGF21 fusion protein comprises an amino acid sequence as shown in SEQ ID NO: 1. In some optional embodiments of the present invention, the GLP-1-Fc-FGF21 fusion protein comprises an amino acid sequence as shown in SEQ ID NO: 2. In some optional embodiments of the present invention, the GLP-1-Fc-FGF21 fusion protein comprises an amino acid sequence as shown in SEQ ID NO: 3. In some optional embodiments of the present invention, the GLP-1-Fc-FGF21 fusion protein comprises an amino acid sequence as shown in SEQ ID NO: 4. In some optional embodiments of the present invention, the GLP-1-Fc-FGF21 fusion protein comprises an amino acid sequence as shown in SEQ ID NO: 5. In some optional embodiments of the present invention, the GLP-1-Fc-FGF21 fusion protein comprises an amino acid sequence as shown in SEQ ID NO: 6. In some optional embodiments of the present invention, the GLP-1-Fc-FGF21 fusion protein comprises an amino acid sequence as shown in SEQ ID NO: 7. In some optional embodiments of the present invention, the GLP-1-Fc-FGF21 fusion protein comprises an amino acid sequence as shown in SEQ ID NO: 8. In some optional embodiments of the present invention, the GLP-1-Fc-FGF21 fusion protein comprises an amino acid sequence as shown in SEQ ID NO: 9. In some optional embodiments of the present invention, the GLP-1-Fc-FGF21 fusion protein comprises an amino acid sequence as shown in SEQ ID NO: 10. In some optional embodiments of the present invention, the GLP-1-Fc-FGF21 fusion protein comprises an amino acid sequence as shown in SEQ ID NO: 11. In some optional embodiments of the present invention, the GLP-1-Fc-FGF21 fusion protein comprises an amino acid sequence as shown in SEQ ID NO: 12.

According to an embodiment of the present invention, the injection comprises: a GLP-1-Fc-FGF21 fusion protein, a histidine buffer, sucrose, and polysorbate 80 or polysorbate 20; the GLP-1-Fc-FGF21 fusion protein comprises an amino acid sequence as shown in SEQ ID NO: 1, and the concentration of the GLP-1-Fc-FGF21 fusion protein in the injection is 25-50 mg/mL; the concentration of the histidine buffer in the injection is 10-20 mM; the concentration of sucrose in the injection is 85-95 mg/mL; the concentration of polysorbate 80 in the injection is 0.4-1.3 mg/mL, or the concentration of polysorbate 20 in the injection is 0.1-0.5 mg/mL; and the pH of the injection is 6.9-7.5.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present application, the concentration of phenol in the composition or injection is 0-7 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, or any range between two of these values as endpoints (e.g., 3-7 mg/mL, 2-4 mg/mL).

In another optional embodiment of the present application, the concentration of m-cresol in the composition or injection is 0-4 mg/mL, for example, 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, or any range between two of these values as endpoints (such as 2-4 mg/mL).

In another aspect of the present invention, it provides an injection. According to an embodiment of the present invention, the injection comprises: a GLP-1-Fc-FGF21 fusion protein, a citrate buffer, sucrose or trehalose, and optionally disodium edetate or calcium disodium edetate, as well as polysorbate 80 or polysorbate 20; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL; the concentration of the citrate buffer in the injection is 8~25 mM, preferably 10~20 mM; the concentration of sucrose in the injection is 85~95 mg/mL; the concentration of trehalose in the injection is 55~65 mg/mL; the concentration of polysorbate 80 in the injection is 0.4~1.3 mg/mL, or the concentration of polysorbate 20 in the injection is 0.1~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present application, the concentration of phenol in the composition or injection is 0-7 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, or any range between two of these values as endpoints (e.g., 3-7 mg/mL, 2-4 mg/mL).

In another optional embodiment of the present application, the concentration of m-cresol in the composition or injection is 0-4 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, or any range between two of these values as endpoints (e.g., 2-4 mg/mL).

In another aspect of the present invention, it provides an injection. According to an embodiment of the present invention, the injection comprises: a GLP-1-Fc-FGF21 fusion protein, a citrate buffer, mannitol, and optionally disodium edetate or calcium disodium edetate, as well as polysorbate 80 or polysorbate 20; wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL; the concentration of the citrate buffer in the injection is 8~25 mM, preferably 10~20 mM; the concentration of mannitol in the injection is 35~45 mg/mL; the concentration of polysorbate 80 in the injection is 0.4~1.5 mg/mL, or 0.4~1.3 mg/mL, or the concentration of polysorbate 20 in the injection is 0.1~0.5 mg/mL; the concentration of the metal chelating agent in the injection is 0.05~0.5 mg/mL; and the pH of the injection is 6.9~7.5. The injection of the invention can effectively control the generation of visible particles, improve the physical and chemical stability of the fusion protein, thereby reducing potential safety risks and extending the shelf life.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present application, the concentration of phenol in the composition or injection is 0-7 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, or any range between two of these values as endpoints (e.g., 3-7 mg/mL, 2-4 mg/mL).

In another optional embodiment of the present application, the concentration of m-cresol in the composition or injection is 0-4 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, or any range between two of these values as endpoints (e.g., 2-4 mg/mL).

In another aspect of the present invention, it provides an injection. According to an embodiment of the present invention, the injection comprises: a GLP-1-Fc-FGF21 fusion protein, a citrate buffer, mannitol, polysorbate 80, and optionally disodium edetate or calcium disodium edetate; wherein the GLP-1-Fc-FGF21 fusion protein has an amino acid sequence as shown in any one of SEQ ID NOs: 1-12, and the concentration of the GLP-1-Fc-FGF21 fusion protein in the injection is 3-25 mg/mL; the concentration of the citrate buffer in the composition is 5-20 mM; the concentration of mannitol in the composition is 40-50 mg/mL; the concentration of polysorbate 80 in the composition is 0.1-1.5 mg/mL; the concentration of the metal chelating agent in the composition is 0.05-0.5 mg/mL; the pH of the injection is 6.4-8.0. The injection of the present invention can effectively control the generation of visible particles and aggregates, improve the physical and chemical stability of the fusion protein, thereby reducing potential safety risks and extending the shelf life.

In some optional embodiments of the present invention, the GLP-1-Fc-FGF21 fusion protein comprises an amino acid sequence as shown in SEQ ID NO: 1. In some optional embodiments of the present invention, the GLP-1-Fc-FGF21 fusion protein comprises an amino acid sequence as shown in SEQ ID NO: 2. In some optional embodiments of the present invention, the GLP-1-Fc-FGF21 fusion protein comprises an amino acid sequence as shown in SEQ ID NO: 3. In some optional embodiments of the present invention, the GLP-1-Fc-FGF21 fusion protein comprises an amino acid sequence as shown in SEQ ID NO: 4. In some optional embodiments of the present invention, the GLP-1-Fc-FGF21 fusion protein comprises an amino acid sequence as shown in SEQ ID NO: 5. In some optional embodiments of the present invention, the GLP-1-Fc-FGF21 fusion protein comprises an amino acid sequence as shown in SEQ ID NO: 6. In some optional embodiments of the present invention, the GLP-1-Fc-FGF21 fusion protein comprises an amino acid sequence as shown in SEQ ID NO: 7. In some optional embodiments of the present invention, the GLP-1-Fc-FGF21 fusion protein comprises an amino acid sequence as shown in SEQ ID NO: 8. In some optional embodiments of the present invention, the GLP-1-Fc-FGF21 fusion protein comprises an amino acid sequence as shown in SEQ ID NO: 9. In some optional embodiments of the present invention, the GLP-1-Fc-FGF21 fusion protein comprises an amino acid sequence as shown in SEQ ID NO: 10. In some optional embodiments of the present invention, the GLP-1-Fc-FGF21 fusion protein comprises an amino acid sequence as shown in SEQ ID NO: 11. In some optional embodiments of the present invention, the GLP-1-Fc-FGF21 fusion protein comprises an amino acid sequence as shown in SEQ ID NO: 12.

In some optional embodiments of the present invention, the concentration of the citrate buffer in the injection is 8-20 mM. In some optional embodiments of the present invention, the concentration of the citrate buffer in the injection is 10-20 mM. In some optional embodiments of the present invention, the concentration of the citrate buffer in the injection is 10-15 mM.

According to an embodiment of the present invention, the injection comprises: a GLP-1-Fc-FGF21 fusion protein, a citrate buffer, mannitol, polysorbate 80, and optionally disodium edetate or calcium disodium edetate; wherein the GLP-1-Fc-FGF21 fusion protein comprises an amino acid sequence as shown in SEQ ID NO: 1, and the concentration of the GLP-1-Fc-FGF21 fusion protein in the injection is 3-25 mg/mL; the concentration of the citrate buffer in the injection is 5-25 mM, 8-20 mM, 10-20 mM, or 10-15 mM; the concentration of mannitol in the injection is 40-50 mg/mL; the concentration of polysorbate 80 in the injection is 0.1-0.5 mg/mL; the concentration of the metal chelating agent in the injection is 0.05-0.5 mg/mL; the pH of the injection is 6.4-8.0.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present application, the concentration of phenol in the composition or injection is 0-7 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, or any range between two of these values as endpoints (e.g., 3-7 mg/mL, 2-4 mg/mL).

In another optional embodiment of the present application, the concentration of m-cresol in the composition or injection is 0-4 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, or any range between two of these values as endpoints (e.g., 2-4 mg/mL).

In another aspect of the present invention, it provides an injection. According to an embodiment of the present invention, the injection comprises: a GLP-1-Fc-FGF21 fusion protein, a citrate buffer, polysorbate 80, optionally disodium edetate or calcium disodium edetate, and sucrose or trehalose; wherein the GLP-1-Fc-FGF21 fusion protein has an amino acid sequence as shown in any one of SEQ ID NOs: 1-12, and the concentration of the GLP-1-Fc-FGF21 fusion protein in the injection is 3-25 mg/mL; the concentration of the citrate buffer in the composition is 5-20 mM; the concentration of sucrose in the composition is 85-95 mg/mL; the concentration of trehalose in the composition is 55-65 mg/mL; the concentration of polysorbate 80 in the composition is 0.1-1.5 mg/mL; the pH of the injection is 6.4-8.0. The injection of the present invention can effectively control the generation of visible particles and aggregates, improve the physical and chemical stability of the fusion protein, thereby reducing potential safety risks and extending the shelf life.

In some optional embodiments of the present invention, the GLP-1-Fc-FGF21 fusion protein comprises an amino acid sequence as shown in SEQ ID NO: 1. In some optional embodiments of the present invention, the GLP-1-Fc-FGF21 fusion protein comprises an amino acid sequence as shown in SEQ ID NO: 2. In some optional embodiments of the present invention, the GLP-1-Fc-FGF21 fusion protein comprises an amino acid sequence as shown in SEQ ID NO: 3. In some optional embodiments of the present invention, the GLP-1-Fc-FGF21 fusion protein comprises an amino acid sequence as shown in SEQ ID NO: 4. In some optional embodiments of the present invention, the GLP-1-Fc-FGF21 fusion protein comprises an amino acid sequence as shown in SEQ ID NO: 5. In some optional embodiments of the present invention, the GLP-1-Fc-FGF21 fusion protein comprises an amino acid sequence as shown in SEQ ID NO: 6. In some optional embodiments of the present invention, the GLP-1-Fc-FGF21 fusion protein comprises an amino acid sequence as shown in SEQ ID NO: 7. In some optional embodiments of the present invention, the GLP-1-Fc-FGF21 fusion protein comprises an amino acid sequence as shown in SEQ ID NO: 8. In some optional embodiments of the present invention, the GLP-1-Fc-FGF21 fusion protein comprises an amino acid sequence as shown in SEQ ID NO: 9. In some optional embodiments of the present invention, the GLP-1-Fc-FGF21 fusion protein comprises an amino acid sequence as shown in SEQ ID NO: 10. In some optional embodiments of the present invention, the GLP-1-Fc-FGF21 fusion protein comprises an amino acid sequence as shown in SEQ ID NO: 11. In some optional embodiments of the present invention, the GLP-1-Fc-FGF21 fusion protein comprises an amino acid sequence as shown in SEQ ID NO: 12.

In some optional embodiments of the present invention, the concentration of the citrate buffer in the injection is 8-20 mM. In some optional embodiments of the present invention, the concentration of the citrate buffer in the injection is 10-20 mM. In some optional embodiments of the present invention, the concentration of the citrate buffer in the injection is 10-15 mM.

According to an embodiment of the present invention, the injection comprises: a GLP-1-Fc-FGF21 fusion protein, a citrate buffer, polysorbate 80, sucrose or trehalose, and optionally disodium edetate or calcium disodium edetate; wherein the GLP-1-Fc-FGF21 fusion protein comprises an amino acid sequence as shown in SEQ ID NO: 1, and the concentration of the GLP-1-Fc-FGF21 fusion protein in the injection is 3-25 mg/mL; the concentration of the citrate buffer in the injection is 5-25 mM, 8-20 mM, 10-20 mM, or 10-15 mM; the concentration of sucrose in the injection is 80-95 mg/mL; the concentration of trehalose in the injection is 55-65 mg/mL; the concentration of polysorbate 80 in the injection is 0.1-0.5 mg/mL; the pH of the injection is 6.4-8.0.

According to an embodiment of the present invention, the injection does not contain disodium edetate or calcium disodium edetate.

In an optional embodiment of the present invention, the injection described in the aforementioned aspects may further include a preservative.

In an optional embodiment of the present invention, the preservative is at least one selected from phenol, m-cresol, benzyl alcohol, and phenoxyethanol.

In an optional embodiment of the present application, the concentration of phenol in the composition or injection is 0-7 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, or any range between two of these values as endpoints (e.g., 3-7 mg/mL, 2-4 mg/mL).

In another optional embodiment of the present application, the concentration of m-cresol in the composition or injection is 0-4 mg/mL, such as 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, or any range between two of these values as endpoints (e.g., 2-4 mg/mL).

### Use

In another aspect of the invention, it provides a use of the aforementioned composition or injection in the preparation of a medicament for treating and/or preventing metabolic diseases.

In some optional embodiments of the present invention, the metabolic diseases include diabetes, obesity, hyperlipidemia, and diseases related to fatty liver.

In some optional embodiments of the present invention, the diseases related to fatty liver include non-alcoholic fatty liver disease (NAFLD), non-alcoholic steatohepatitis (NASH), liver fibrosis, and cirrhosis.

### Method for Treating and/or Preventing Metabolic Diseases

In another aspect of the present invention, a method for treating and/or preventing metabolic diseases is proposed. According to an embodiment of the present invention, the method comprises administering to a subject a pharmaceutically acceptable amount of the aforementioned composition or the aforementioned injection.

The effective amount of the composition or injection described in the present invention may vary depending on the mode of administration, the severity of the disease to be treated, and other factors. The selection of a preferred effective amount can be determined by those skilled in the art based on various factors (for example, through clinical trials). These factors include, but are not limited to, pharmacokinetic parameters of the active ingredient such as bioavailability, metabolism, half-life, etc.; the severity of the disease to be treated, the patient's body weight, the patient's immune status, the route of administration, and the like. For example, depending on the urgency of the treatment condition, administration may be performed at a dosage of once daily, once every other day, once weekly, or once every two weeks.

In some optional embodiments of the present invention, the route of administration of the method includes subcutaneous injection or intravenous injection, preferably subcutaneous injection.

### Injection Device or Container

In another aspect of the invention, it provides an injection device or container. According to an embodiment of the present invention, the injection device or container comprises: the aforementioned composition or the aforementioned injection.

In some optional embodiments of the present invention, the injection device or container comprises at least one selected from a vial, a bollte, a cartridge, and a syringe.

In some optional embodiments of the present invention, the syringe comprises at least one selected from an auto-injector and a prefilled syringe.

The following examples will explain the solutions of the present invention in conjunction with specific embodiments. Those skilled in the art will understand that the following examples are only intended to illustrate the present invention and should not be construed as limiting the scope of the invention. In the examples, specific techniques or conditions not indicated are carried out according to the techniques or conditions described in the literature in the field or according to the product manual. Reagents or instruments not specified by the manufacturer are conventional products that can be obtained commercially.

It should be noted that the amino acid sequence of the GLP-1-Fc-FGF21 dual-target fusion protein in Example 1 of the present invention is as shown in SEQ ID NO: 1, and it is prepared using conventional methods in the field.

### Example 1

### 1. Investigation of Injections Prepared with Different Buffer Systems

1.1 Injections of low concentration formulations A1~A3 were prepared using different buffer systems. The final concentration of the GLP-1-Fc-FGF21 dual-target fusion protein (referred to as the fusion protein, i.e., API) and the types and final concentrations of excipients in the injections are detailed in Table 1. The preparation method for the injections was as follows: excipients and API were weighed and mixed according to the formulation amounts listed in Table 1. If necessary, the pH of the formulation was adjusted to the corresponding value using 2% hydrochloric acid solution or 2% sodium hydroxide solution. Finally, the solution was adjusted to volume with water for injection. After preparation, the drug solution was filtered through 0.45 µm and 0.22 µm PVDF membranes in series into 1 ml slender pre-filled syringes, with a fill volume of 1 ml per syringe, and then sealed with rubber stoppers. Subsequently, formulations A1~A3 were subjected to high-temperature stability studies. The stability test results using SEC-HPLC after storage at 40°C for 0 days, 10 days, and 14 days are shown in Table 2.

**Table 1: Composition ratios of components in formulations A1~A3.**

| Formulation | Concentration of GLP-1-Fc-FGF2 1 Dual-Target Fusion Protein (mg/ml) | Buffer System and Its Amount | Mannitol (mg/ml) | Polysorbate 80 (mg/ml) | pH Value |
|---|---|---|---|---|---|
| A1 | 2mg/ml | Citric Acid Monohydrate (0.14 mg/ml), Sodium Citrate Dihydrate (2.74 mg/ml), i.e., 10 mM | 46.4 | 0.2 | 6.5 |
| A2 | 2mg/ml | Sodium Dihydrogen Phosphate Monohydrate (0.846 mg/ml), Disodium Hydrogen Phosphate Anhydrous (0.549 mg/ml), i.e., Phosphate Buffer 10 mM | 46.4 | 0.2 | 6.5 |
| A3 | 2mg/ml | Histidine (1.552 mg/ml, i.e., 10 mM), Hydrochloric Acid (0.324 mg/ml) | 46.4 | 0.2 | 6.5 |

**Table 2: Stability Results of Formulations A1~A3 under High-Temperature Conditions at 40°C**

| Test Item | Formulation | High Temperature 40°C | | |
|---|---|---|---|---|
| | | 0 day | 10 day | 14 day |
| SEC-Purity (Monomer Including Shoulder Peaks) (%) | A1 | 96.91 | 97.42 | 96.92 |
| | A2 | 96.64 | 96.54 | 96.33 |
| | A3 | 97.84 | 96.20 | 95.51 |
| SEC-Purity (Aggregates) (%) | A1 | 2.21 | 1.00 | 1.27 |
| | A2 | 2.62 | 1.08 | 1.07 |
| | A3 | 1.50 | 1.30 | 1.38 |
| RP-HPLC-Purity (Main Peak) (%) | A1 | 72.17 | 60.74 | 56.30 |
| | A2 | 72.58 | 51.97 | 46.69 |
| | A3 | 74.58 | 52.64 | 42.14 |
| RP-HPLC-Purity (Total Related Substances) (%) | A1 | 27.83 | 39.26 | 43.70 |
| | A2 | 27.42 | 48.03 | 53.31 |
| | A3 | 25.42 | 47.36 | 57.86 |
| Visible Particles* | A1 | Complies with Specifications | Complies with Specifications | Complies with Specifications |
| | A2 | Complies with Specifications | Complies with Specifications | Does Not Comply with Specifications |
| | A3 | Complies with Specifications | Complies with Specifications | Does Not Comply with Specifications |

| | | | | |
|---|---|---|---|---|
| Note: (1) The "*" in the table above and throughout the text indicates that the visible particles comply with the requirements of ChP 2015 Edition, Part IV, General Rule 0904. Visible particles not complying with specifications means they do not meet the requirements of ChP 2015 Edition, Part IV, General Rule 0904. (2) The lower main peak purity in RP-HPLC at 0 days in the table above is primarily due to the low main peak purity of the API stock solution directly used in this example. Therefore, the data in this example are only used for screening formulation and do not represent the actual purity of the fusion protein samples in this application, as is the case with formulations A4~A37 in this example. | | | | |

From Table 2, it can be observed that the citrate buffer system exhibits the slowest rate of decline in RP-HPLC main peak purity, and visible particles comply with specifications after 14 days at 40°C. Therefore, for low-concentration fusion protein formulations, the citrate buffer system demonstrates relatively better chemical and physical stability.

Since different buffer systems may also have varying effects on the thermal stability of proteins, further investigation was conducted using Differential Scanning Calorimetry (DSC) to determine the Tm and Tonset values of formulations A1~A3 in different buffer systems. This was done to study and compare the effects of various factors on the thermal stability of proteins based on temperature changes during protein denaturation. The DSC test results are shown in Table 3. Here, Tonset and Tm values are indicators of thermal stability. The Tonset value represents the initial temperature at which protein unfolding begins, while the Tm value is the midpoint temperature of the protein thermal transition. Generally, the higher the Tonset and Tm values, the more stable the protein, and the less likely it is to unfold or denature in low-temperature environments.

**Table 3: DSC Test Results of Formulations A1~A3**

| Formulation | pH | Tₒₙₛₑₜ (°C) | Tₘ₁ (°C) | Tₘ₂ (°C) |
|---|---|---|---|---|
| A1 | 6.5 | 60.92 | 66.78 | 84.40 |
| A2 | 6.5 | 60.23 | 66.64 | 83.71 |
| A3 | 6.5 | 56.36 | 64.39 | 83.91 |

From Table 3, it can be seen that by comparing the Tonset, Tm1, and Tm2 values, the thermal stability of low-concentration fusion protein formulations in citrate (Formulation A1) and phosphate buffer (Formulation A2) is superior to that in the histidine buffer system (Formulation A3).

1.2 Preparation of high-concentration injection F1~F4 using different buffer systems, the final concentration of the GLP-1-Fc-FGF21 dual-target fusion protein (referred to as the fusion protein) and the types and final concentrations of excipients in the injections are detailed in Table 4. The preparation method for the injections was as follows: The fusion protein stock solution was ultrafiltered and concentrated, followed by buffer exchange. After measuring the protein concentration, the fusion protein solution was diluted to 25 mg/ml using the corresponding buffer system. If necessary, the pH was adjusted to 7.0 using 2% hydrochloric acid or 2% sodium hydroxide solution. After preparation, the drug solution was filtered through 0.45 µm and 0.22 µm PVDF membranes in series into 2R borosilicate glass vials, with a fill volume of 1 ml per vial. The vials were sealed with rubber stoppers and aluminum-plastic caps. Subsequently, formulations F1~F4 were subjected to high-temperature stability studies. The rate of change in SEC-HPLC aggregates (%/day) after storage at 40°C for 14 days is shown in Tables 5-1, 5-2, and 5-3.

**Table 4: Ratios of Components in Formulations F1~F4**

| Formulation | GLP-1-Fc-FGF21 Dual-Target Fusion Protein Concentration (mg/ml) | Buffer System Type | Buffer System Amount | pH |
|---|---|---|---|---|
| F1 | 25 | Sodium Citrate | 20mM | 7.0 |
| F2 | 25 | Histidine | 20mM | 7.0 |
| F3 | 25 | Disodium Hydrogen Phosphate Anhydrous, Sodium Dihydrogen Phosphate Monohydrate | 20mM | 7.0 |
| F4 | 25 | Tris | 20mM | 7.0 |
| F54 | 40 | Citric Acid Monohydrate, Sodium Citrate Dihydrate | 10 mM | 7.0 |

**Table 5-1: Rate of Change in SEC-HPLC Aggregates (%/day) for Formulations F1~F4 after 14 Days at 40°C**

| Formulation | 0 Days - SEC Aggregates (%) | 14 Days at 40°C - SEC Aggregates (%) | Rate of Change (%/day) |
|---|---|---|---|
| F1 | 1.59 | 4.27 | 0.19 |
| F2 | 1.06 | 1.63 | 0.04 |
| F3 | 1.57 | 3.72 | 0.15 |
| F4 | 1.55 | 3.87 | 0.17 |
| F54 | 2.46 | 4.14 | 0.12 |

**Table 5-2: Rate of Change in IEX-HPLC Main Peak (%/day) for Formulations F1~F4 after 14 Days at 40°C**

| Formulation | 0 Days - IEX Main Peak (%) | 14 Days at 40°C - IEX Main Peak (%) | Rate of Change (%/day) |
|---|---|---|---|
| F1 | 58.69 | 41.02 | 1.26 |
| F2 | 56.90 | 42.56 | 1.02 |
| F3 | 57.04 | 35.91 | 1.51 |
| F4 | 55.73 | 43.10 | 0.90 |

**Table 5-3: Rate of Change in RP-HPLC Total Impurities (%/day) for Formulations F1~F4 after 14 Days at 40°C**

| Formulation | 0 Days - RP-HPLC Total Impurities (%) | 14 Days at 40°C - RP-HPLC | Rate of Change (%/day) |
|---|---|---|---|
| F1 | 21.80 | 31.06 | 0.66 |
| F2 | 22.30 | 33.99 | 0.83 |
| F3 | 20.54 | 31.34 | 0.77 |
| F4 | 22.18 | 30.64 | 0.60 |

From Table 5-1, it can be observed that after 14 days at 40°C, the growth rate of SEC aggregates was relatively faster in the sodium citrate buffer (Formulation F1), phosphate buffer (Formulation F3), and Tris buffer (Formulation F4) systems. In contrast, the histidine buffer (Formulation F2) significantly reduced the growth rate of SEC aggregates (the SEC aggregate standard is 5%). For RP-HPLC total impurities (total impurity standard is 40%), the rate of change was comparable across all four formulations. Therefore, it can be further concluded that when the concentration of the fusion protein is greater than or equal to 25 mg/ml, the formulation prepared with the histidine buffer exhibits higher stability. However, the stability data for formulations with different buffer systems all fall within acceptable standard limits.

### 2. Investigation of Injections Prepared at Different pH Levels

2.1 Injections of low concentration formulations A4~A8 were prepared using different pH values. The final concentration of the GLP-1-Fc-FGF21 dual-target fusion protein (referred to as the fusion protein, i.e., API) and the types and final concentrations of excipients in the injections are detailed in Table 6. The preparation method for the injections was as follows: Excipients and API were weighed according to the formulation amounts listed in Table 6 and mixed. If necessary, the pH of the formulation was adjusted to the corresponding value using 2% hydrochloric acid solution or 2% sodium hydroxide solution. Finally, the solution was adjusted to volume with water for injection. After preparation, the drug solution was filtered through 0.45 µm and 0.22 µm PVDF membranes in series into 1 ml slender pre-filled syringes, with a fill volume of 1 ml per syringe, and then sealed with rubber stoppers. Subsequently, formulations A4~A8 were subjected to high-temperature stability studies and long-term stability studies. The stability test results using SEC-HPLC after storage at 40°C for 0 days, 10 days, and 14 days are shown in Table 7. The stability test results using SEC-HPLC after storage in a 4°C refrigerator for 0 months, 1 month, 6 months, and 12 months are shown in Table 8.

**Table 6: Ratios of Components in Formulations A4~A8**

| Formulation | Concentration of GLP-1-Fc-FGF 21 Dual-Target Fusion Protein (mg/ml) | Buffer System and Its Amount (10 mM) | Mannitol (mg/ml) | Polysorbate 80 (mg/ml) | pH Value |
|---|---|---|---|---|---|
| A4 | 2 | Citric Acid Monohydrate (0.14 mg/ml), Sodium Citrate Dihydrate (2.74 mg/ml) | 46.4 | 0.2 | 6.0 |
| A5 | 2 | Citric Acid Monohydrate (0.14 mg/ml), Sodium Citrate Dihydrate (2.74 mg/ml) | 46.4 | 0.2 | 6.5 |
| A6 | 2 | Citric Acid Monohydrate (0.14 mg/ml), Sodium Citrate Dihydrate (2.74 mg/ml) | 46.4 | 0.2 | 7.0 |
| A7 | 2 | Citric Acid Monohydrate (0.14 mg/ml), Sodium Citrate Dihydrate (2.74 mg/ml) | 46.4 | 0.2 | 7.5 |
| A8 | 2 | Citric Acid Monohydrate (0.14 mg/ml), Sodium Citrate Dihydrate (2.74 mg/ml) | 46.4 | 0.2 | 8.0 |

**Table 7: Stability Results of Formulations A4~A8 under High-Temperature Conditions at 40°C**

| Test Item | Formulation | High Temperature 40°C | | |
|---|---|---|---|---|
| | | 0 days | 7 days | 14 days |
| SEC-Purity (Monomer Including Shoulder Peaks) (%) | A4 | 94.01 | 95.53 | 94.67 |
| | A5 | 94.77 | 97.32 | 96.87 |
| | A6 | 95.01 | 97.92 | 97.17 |
| | A7 | 95.40 | 97.94 | 97.48 |
| | A8 | 94.99 | 97.81 | 97.42 |
| SEC-Purity (Aggregates) (%) | A4 | 5.91 | 4.29 | 4.52 |
| | A5 | 5.23 | 2.53 | 2.42 |
| | A6 | 4.91 | 1.91 | 2.05 |
| | A7 | 4.60 | 1.85 | 1.65 |
| | A8 | 4.98 | 1.94 | 1.65 |
| RP-HPLC-Purity (Main Peak + Peak 4) (%) | A4 | 73.79 | 68.07 | 61.59 |
| | A5 | 72.64 | 70.38 | 66.27 |
| | A6 | 75.26 | 71.14 | 67.71 |
| | A7 | 72.71 | 70.99 | 69.28 |
| | A8 | 74.36 | 71.69 | 67.83 |
| RP-HPLC-Purity (Total Related Substances) (%) | A4 | 26.21 | 31.93 | 38.41 |
| | A5 | 27.36 | 29.62 | 33.73 |
| | A6 | 24.74 | 28.86 | 32.29 |
| | A7 | 27.29 | 29.01 | 30.72 |
| | A8 | 25.64 | 28.31 | 32.17 |
| Visible Particles* | A4 | Complies with Specifications | Complies with Specifications | Does Not Comply with Specifications |
| | A5 | Complies with Specifications | Complies with Specifications | Does Not Comply with Specifications |
| | A6 | Complies with Specifications | Complies with Specifications | Complies with Specifications |
| | A7 | Complies with Specifications | Complies with Specifications | Does Not Comply with Specifications |
| | A8 | Complies with Specifications | Complies with Specifications | Does Not Comply with Specifications |

**Table 8: Stability Results of Formulations A4~A8 at 4°C**

| Test Item | Formulati on | Long-Term Storage at 4°C | | | |
|---|---|---|---|---|---|
| | | 0 months | 1 months | 6 months | 12 months |
| SEC-Purity (Monomer Including Shoulder Peaks) (%) | A4 | 94.01 | 94.09 | 93.58 | 92.93 |
| | A5 | 94.77 | 94.64 | 94.61 | 94.64 |
| | A6 | 95.01 | 94.94 | 95.13 | 95.02 |
| | A7 | 95.40 | 95.12 | 95.26 | 95.14 |
| | A8 | 94.99 | 95.14 | 95.20 | 95.00 |
| SEC-Purity (Aggregates ) (%) | A4 | 5.91 | 5.55 | 5.33 | 5.91 |
| | A5 | 5.23 | 5.02 | 4.28 | 4.16 |
| | A6 | 4.91 | 4.66 | 3.69 | 3.56 |
| | A7 | 4.60 | 4.50 | 3.47 | 3.20 |
| | A8 | 4.98 | 4.48 | 3.44 | 3.12 |
| RP-HPLC-P urity (Main Peak + Peak 4) (%) | A4 | 73.79 | 75.80 | 69.84 | 66.03 |
| | A5 | 72.64 | 74.96 | 66.81 | 66.74 |
| | A6 | 75.26 | 74.28 | 68.32 | 64.97 |
| | A7 | 72.71 | 76.21 | 67.47 | 63.52 |
| | A8 | 74.36 | 74.66 | 66.39 | 61.76 |
| RP-HPLC-P urity (Total Related Substances) (%) | A4 | 26.21 | 24.20 | 30.16 | 33.97 |
| | A5 | 27.36 | 25.04 | 33.19 | 33.26 |
| | A6 | 24.74 | 25.72 | 31.68 | 35.03 |
| | A7 | 27.29 | 23.79 | 32.53 | 36.48 |
| | A8 | 25.64 | 25.34 | 33.61 | 38.24 |
| Visible Particles* | A4 | Complies with Specifications | Complies with Specifications | Complies with Specifications | Does Not Comply with Specifications |
| | A5 | Complies with Specifications | Complies with Specifications | Complies with Specifications | Complies with Specifications |
| | A6 | Complies with Specifications | Complies with Specifications | Complies with Specifications | Complies with Specifications |
| | A7 | Complies with Specifications | Complies with Specifications | Complies with Specifications | Complies with Specifications |
| | A8 | Complies with Specifications | Complies with Specifications | Complies with Specifications | Complies with Specifications |

From Tables 7 and 8, it can be concluded that at pH 6.0, the injection exhibited the fastest decline in RP-HPLC main peak purity, the highest dimer content, and the poorest stability under high-temperature conditions at 40°C. Additionally, after 12 months of long-term storage, visible particles did not comply with specifications, the SEC purity (monomer including shoulder peaks) declined the fastest, the dimer content was the highest, and the stability was the poorest. In contrast, formulations at other pH levels demonstrated good chemical stability.

Since different buffer systems may also have varying effects on the thermal stability of proteins, further investigation was conducted using Differential Scanning Calorimetry (DSC) to determine the Tm and Tonset values of formulations A4~A8 in different buffer systems. This was done to study and compare the effects of various factors on the thermal stability of proteins based on temperature changes during protein denaturation. The DSC test results are shown in Table 9.

**Table 9: DSC Test Results of Formulations A4~A8**

| Formulation | pH | Tonset (°C) | Tm1 (°C) | Tm2 (°C) |
|---|---|---|---|---|
| A4 | 6.0 | 57.86 | 64.79 | 84.29 |
| A5 | 6.5 | 60.62 | 66.49 | 84.37 |
| A6 | 7.0 | 60.78 | 67.45 | 84.00 |
| A7 | 7.5 | 59.92 | 67.94 | 83.69 |
| A8 | 8.0 | 60.35 | 67.84 | 83.58 |

From the DSC test results in Table 9, it can be observed that the thermal stability is the poorest at pH 6.0, while the other pH groups exhibit relatively better stability.

To further investigate the impact of pH on product stability within the range of 6.0 to 7.0. Formulations A9~A15 were prepared at different pH levels. The final concentration of the GLP-1-Fc-FGF21 dual-target fusion protein (referred to as the fusion protein) and the types and final concentrations of excipients in the injections are detailed in Table 10. The preparation method for the injections follows the steps described in Section 2.1 of Example 1 for preparing formulations A4~A8. Subsequently, formulations A9~A15 were subjected to high-temperature stability studies and DSC testing. The stability test results using SEC-HPLC after storage at 40°C for 0 days, 10 days, 14 days, and 21 days are shown in Table 11. The DSC test results are shown in Table 12.

**Table 10: Ratios of Components in Formulations A9~A15**

| Formu lation | Concentration of GLP-1-Fc-FGF 21 Dual-Target Fusion Protein (mg/ml) | Buffer System and Its Amount (10 mM) | Mannitol (mg/ml) | Polysorbat e 80 (mg/ml) | pH Value |
|---|---|---|---|---|---|
| A9 | 3 | Citric Acid Monohydrate (0.14 mg/ml), Sodium Citrate Dihydrate (2.74 mg/ml) | 46.4 | 0.2 | 6.0 |
| A10 | 3 | Citric Acid Monohydrate (0.14 mg/ml), Sodium Citrate Dihydrate (2.74 mg/ml) | 46.4 | 0.2 | 6.2 |
| A11 | 3 | Citric Acid Monohydrate (0.14 mg/ml), Sodium Citrate Dihydrate (2.74 mg/ml) | 46.4 | 0.2 | 6.4 |
| A12 | 3 | Citric Acid Monohydrate (0.14 mg/ml), Sodium Citrate Dihydrate (2.74 mg/ml) | 46.4 | 0.2 | 6.5 |
| A13 | 3 | Citric Acid Monohydrate (0.14 mg/ml), Sodium Citrate Dihydrate (2.74 mg/ml) | 46.4 | 0.2 | 6.6 |
| A14 | 3 | Citric Acid Monohydrate (0.14 mg/ml), Sodium Citrate Dihydrate (2.74 mg/ml) | 46.4 | 0.2 | 6.8 |
| A15 | 3 | Citric Acid Monohydrate (0.14 mg/ml), Sodium Citrate Dihydrate (2.74 mg/ml) | 46.4 | 0.2 | 7.0 |

**Table 11: Stability Results of Formulations A9~A15 under High-Temperature Conditions at 40°C**

| Test Items | Formulati on | High Temperature 40°C | | | |
|---|---|---|---|---|---|
| | | 0 days | 7 days | 14 days | 21 days |
| SEC-Purit y (Monomer Including Shoulder Peaks) (%) | A9 | 98.53 | 96.55 | 95.60 | 94.51 |
| | A10 | 98.76 | 97.48 | 97.69 | 96.92 |
| | A11 | 98.84 | 98.30 | 98.33 | 97.70 |
| | A12 | 98.96 | 98.52 | 98.49 | 98.44 |
| | A13 | 98.94 | 98.61 | 98.86 | 98.58 |
| | A14 | 99.07 | 98.68 | 98.84 | 98.40 |
| | A15 | 99.05 | 98.92 | 98.91 | 98.45 |
| SEC-Purit y (Aggregat es) (%) | A9 | 1.42 | 3.33 | 3.96 | 4.89 |
| | A10 | 1.21 | 2.25 | 2.26 | 2.59 |
| | A11 | 1.16 | 1.52 | 1.58 | 1.86 |
| | A12 | 0.98 | 1.30 | 1.24 | 1.41 |
| | A13 | 1.00 | 1.28 | 1.12 | 1.32 |
| | A14 | 0.93 | 1.08 | 0.95 | 1.11 |
| | A15 | 0.87 | 0.86 | 0.84 | 0.98 |
| RP-HPLC-Purity (Main Peak + Peak 4) (%) | A9 | 79.70 | 71.50 | 68.83 | 57.11 |
| | A10 | 80.87 | 73.32 | 71.48 | 62.33 |
| | A11 | 81.86 | 73.89 | 72.21 | 68.16 |
| | A12 | 83.53 | 75.84 | 73.67 | 68.29 |
| | A13 | 79.84 | 74.58 | 71.33 | 67.68 |
| | A14 | 79.51 | 76.35 | 74.05 | 69.42 |
| | A15 | 79.41 | 75.29 | 74.36 | 71.17 |
| RP-HPLC -Purity (Total Related Substance s) (%) | A9 | 20.30 | 28.51 | 31.18 | 42.89 |
| | A10 | 19.13 | 26.68 | 28.52 | 37.67 |
| | A11 | 18.14 | 26.11 | 27.79 | 31.84 |
| | A12 | 16.48 | 24.16 | 26.33 | 31.71 |
| | A13 | 20.16 | 25.42 | 28.67 | 32.32 |
| | A14 | 20.49 | 23.65 | 25.95 | 30.58 |
| | A15 | 20.59 | 24.71 | 25.64 | 28.83 |
| Visible Particles* | A9 | Complies with Specifications | Does Not Comply with Specifications | Does Not Comply with Specifications | Does Not Comply with Specifications |
| | A10 | Complies with Specifications | Complies with Specifications | Complies with Specifications | Does Not Comply with Specifications |
| | A11 | Complies with Specifications | Complies with Specifications | Complies with Specifications | Complies with Specifications |
| | A12 | Complies with Specifications | Complies with Specifications | Complies with Specifications | Complies with Specifications |
| | A13 | Complies with Specifications | Complies with Specifications | Complies with Specifications | Complies with Specifications |
| | A14 | Complies with Specifications | Complies with Specifications | Complies with Specifications | Complies with Specifications |
| | A15 | Complies with Specifications | Complies with Specifications | Complies with Specifications | Complies with Specifications |

**Table 12: DSC Test Results for Formulations A9~A15**

| Formulation | pH | Tₒₙₛₑₜ (°C) | Tₘ₁ (°C) | Tm2 (°C) |
|---|---|---|---|---|
| A9 | 6.0 | 58.96 | 64.57 | 84.31 |
| A10 | 6.2 | 59.04 | 65.45 | 84.39 |
| A11 | 6.4 | 60.36 | 66.50 | 84.37 |
| A12 | 6.5 | 60.24 | 66.65 | 84.26 |
| A13 | 6.6 | 60.81 | 66.94 | 84.29 |
| A14 | 6.8 | 59.93 | 67.40 | 83.94 |
| A15 | 7.0 | 59.80 | 67.55 | 84.09 |

From Tables 11 and 12, it can be observed that at pH values of 6.0 and 6.2, both the Tonset and Tm1 values are relatively low, indicating poorer thermal stability compared to the other groups. However, at pH values of 6.4 and above, the chemical stability, physical stability, and thermal stability of the injection are all better.

2.2 Injections of high-concentration injections F5~F11 were prepared using different pH values. The final concentration of the GLP-1-Fc-FGF21 dual-target fusion protein (referred to as the fusion protein, or API) and the types and final concentrations of the excipients in the injection are detailed in Table 13. The preparation method for the injection is as follows: the excipients and API were weighed and mixed according to the formulation table. If necessary, the pH of the formulation was adjusted to the desired value using a 2% hydrochloric acid solution or a 2% sodium hydroxide solution. Finally, the mixture was diluted with water for injection to a total volume of 10 ml. After preparation, the solution was filtered through 0.45 µm and 0.22 µm PVDF membranes in series into 1 ml long thin pre-filled syringes, with a fill volume of 0.5 ml per syringe, and then sealed with rubber stoppers. Then, conduct high-temperature stability studies on formulations F1~F4. The rate of change in SEC-HPLC aggregates (day/%) after placing the formulations at 40°C for 20 days is shown in Table 14.

**Table 13: Ratios of Components in Formulations F5~F11.**

| Formulation | GLP-1-Fc-FGF21 Dual-Target Fusion Protein Concentration (mg/ml) | Histidine Hydrochloride | Mannitol (mg/ml) | Polysorbate 80 (mg/ml) | pH |
|---|---|---|---|---|---|
| F5 | 40 | 10mM | 46.4 | 0.5 | 6.6 |
| F6 | 40 | 10mM | 46.4 | 0.5 | 6.7 |
| F7 | 40 | 10mM | 46.4 | 0.5 | 6.9 |
| F8 | 40 | 10mM | 46.4 | 0.5 | 7.0 |
| F9 | 40 | 10mM | 46.4 | 0.5 | 7.1 |
| F10 | 40 | 10mM | 46.4 | 0.5 | 7.3 |
| F11 | 40 | 10mM | 46.4 | 0.5 | 7.5 |

**Table 14: Rate of Change in SEC-HPLC Aggregates (Day/%) for Formulations F5~F11 After Storage at 40°C for 20 Days**

| Formulation | 0 Days - SEC Aggregates (%) | 20 Days at 40°C - SEC Aggregates (%) | Rate of Change (%/day) |
|---|---|---|---|
| F5 | 1.46 | 5.53 | 0.20 |
| F6 | 1.44 | 4.96 | 0.18 |
| F7 | 1.01 | 3.74 | 0.14 |
| F8 | 0.89 | 2.76 | 0.09 |
| F9 | 0.93 | 2.55 | 0.08 |
| F10 | 0.88 | 2.21 | 0.07 |
| F11 | 0.86 | 2.06 | 0.06 |

From Table 14, it can be observed that after storage at 40°C for 20 days, within the pH range of 6.6-7.5, the increase in SEC aggregates slows down as the pH increases. At pH 6.6, the high-molecular-weight protein increased to 5.53% by day 20, exceeding the established quality standard limit of 5%. At pH values of 6.7-7.5, the rate of SEC aggregate growth remained below 5%.

Furthermore, formulations F5-F11 were subjected to room temperature shaking studies for 72 hours at a shaking speed of 600 rpm. The results of the shaking experiment are shown in Table 15 below.

**Table 15: Rate of Change in SEC-HPLC Aggregates (Day/%) for Formulations F5~F11 After Room Temperature Shaking for 72 Hours**

| Formulation | 0h-SEC Aggregates (%) | SEC Aggregates After 72h Shaking at Room Temperature (%) | Change (%) |
|---|---|---|---|
| F5 | 1.46 | 2.03 | 0.57 |
| F6 | 1.44 | 1.96 | 0.52 |
| F7 | 1.01 | 1.42 | 0.41 |
| F8 | 0.89 | 1.33 | 0.44 |
| F9 | 0.93 | 1.30 | 0.37 |
| F10 | 0.88 | 1.22 | 0.34 |
| F11 | 0.86 | 1.17 | 0.31 |

From Table 15, it can be observed that after shaking for 72 hours, the SEC aggregates in formulations F5-F11 showed only a slight increase, and the extent of this increase slowed as the pH value increased. When the formulation pH was in the range of 6.6-7.5, it effectively prevented the formation of excessive protein aggregates caused by shaking.

To further evaluate the impact of different pH values on the conformational stability and colloidal stability of the protein, the Prometheus NT.48 protein stability analyzer from NanoTemper was used to test formulations F5-F11 using the ThermControl module. This analysis provided the protein's denaturation temperature (Tm and Tonset) and the onset temperature for protein aggregation (Tagg). The test results are shown in Table 16 below.

**Table 16: Tagg and Tm Values for Formulations F5~F11**

| Formulation | Tagg (°C) | Tonset (°C) | Tm1 (°C) | Tm2 (°C) |
|---|---|---|---|---|
| F5 | 62.3 | 54.9 | 62.0 | 70.9 |
| F6 | 62.5 | 55.1 | 62.5 | 80.6 |
| F7 | 64.4 | 56.4 | 63.7 | 80.2 |
| F8 | No Aggregation Detected | 57.1 | 64.2 | 80.3 |
| F9 | No Aggregation Detected | 57.0 | 64.5 | 80.4 |
| F10 | No Aggregation Detected | 58.0 | 65.0 | 80.4 |
| F11 | No Aggregation Detected | 58.5 | 65.5 | 80.3 |

From Table 16, it can be observed that at a pH of 6.6, the values of Tonset, Tm, and Tagg are relatively low compared to the other groups, indicating poorer stability. At pH values of 6.7-7.5, as the pH increases, the values of Tonset and Tm1 also increase, indicating better thermal stability. At pH values of 7.0-7.5, no aggregation temperature (Tagg) was detected, suggesting good colloidal stability.

In summary, when the formulation pH is in the range of 6.7-7.5, both the chemical and physical stability of the formulation are optimal.

### 3. Evaluation of Injections Prepared with Different Surfactants

3.1 Preparation of low-concentration injection formulations A16~A19 with different surfactants, the final concentration of the GLP-1-Fc-FGF21 dual-target fusion protein (referred to as the fusion protein or API) and the types and final concentrations of excipients in the injections are detailed in Table 17. The preparation method for the injections is as follows: the excipients and API were weighed and mixed according to the formulation table. If necessary, the pH of the formulation was adjusted to the desired value using a 2% hydrochloric acid solution or a 2% sodium hydroxide solution. Finally, the mixture was diluted with water for injection to the specified volume. After preparation, the solution was filtered through 0.45 µm and 0.22 µm PVDF membranes in series into 1 ml long thin pre-filled syringes, with a fill volume of 0.8 ml per syringe, and then sealed with rubber stoppers. Formulations A16~A19 were subjected to high-temperature stability studies. The SEC-HPLC stability test results after storage at 40°C for 0 days and 10 days are shown in Table 18, the SEC-HPLC stability test results after storage at 40°C for 10 days are shown in Table 19.

**Table 17: Ratios of Components in Formulations A16~A19**

| Formulation | Concentration of GLP-1-Fc-FGF21 Dual-Target Fusion Protein (mg/ml) | Buffer System and Its Amount (10 mM) | Surfactant Types and Concentrations | Mannitol (mg/ml) | pH Value |
|---|---|---|---|---|---|
| A16 | 3 | Citric Acid Monohydrate (0.14 mg/ml), Sodium Citrate Dihydrate (2.74 mg/ml) | Polysorbate 80 (0.2 mg/mL) | 46.4 | 6.5 |
| A17 | 3 | Citric Acid Monohydrate (0.14 mg/ml), Sodium Citrate Dihydrate (2.74 mg/ml) | Polysorbate 20 (0.2 mg/mL) | 46.4 | 6.5 |
| A18 | 3 | Citric Acid Monohydrate (0.14 mg/ml), Sodium Citrate Dihydrate (2.74 mg/ml) | Poloxamer 188 (8mg/ml) | 46.4 | 6.5 |
| A19 | 3 | Citric Acid Monohydrate (0.14 mg/ml), Sodium Citrate Dihydrate (2.74 mg/ml) | Poloxamer 407 (8mg/ml) | 46.4 | 6.5 |

**Table 18: Stability Results for Formulations A16~A19 Under High-Temperature Conditions (40°C)**

| Test Item | Formulation | Surfactant Type | High Temperature 40°C | |
|---|---|---|---|---|
| | | | 0 days | 10 days |
| SEC-Purity (Monomer Including Shoulder Peaks) (%) | A16 | Polysorbate 80 | 99.07 | 98.81 |
| | A17 | Polysorbate 20 | 99.07 | 98.92 |
| | A18 | Poloxamer 188 | 99.20 | 98.98 |
| | A19 | Poloxamer 407 | 98.08 | 97.90 |
| SEC-Purity Test Item (Aggregates) (%) | A16 | Polysorbate 80 | 0.93 | 1.19 |
| | A17 | Polysorbate 20 | 0.93 | 1.08 |
| | A18 | Poloxamer 188 | 0.80 | 0.90 |
| | A19 | Poloxamer 407 | 0.82 | 0.99 |
| RP-HPLC-Pu rity (Main Peak + Peak 4) (%) | A16 | Polysorbate 80 | 77.38 | 76.95 |
| | A17 | Polysorbate 20 | 75.55 | 70.71 |
| | A18 | Poloxamer 188 | 76.69 | 68.22 |
| | A19 | Poloxamer 407 | 77.77 | 60.80 |
| RP-HPLC-Pu rity (Total Related Substances) (%) | A16 | Polysorbate 80 | 22.62 | 23.05 |
| | A17 | Polysorbate 20 | 24.45 | 29.29 |
| | A18 | Poloxamer 188 | 23.31 | 31.78 |
| | A19 | Poloxamer 407 | 22.23 | 39.20 |
| Visible Particles* | A16 | Polysorbate 80 | Complies with Specifications | Complies with Specifications |
| | A17 | Polysorbate 20 | Complies with Specifications | Complies with Specifications |
| | A18 | Poloxamer 188 | Complies with Specifications | Complies with Specifications |
| | A19 | Poloxamer 407 | Complies with Specifications | Does Not Comply with Specifications |

**Table 19: Rate of Change in Various Indicators for Formulations A16~A19 Under High-Temperature Conditions (40°C)**

| Formulation | Surfactant Type | SEC Purity (Monomer including Shoulder Peaks) Daily Decrease Rate (%/day) | RP-HPLC Purity (Main Peak + Peak 4) Daily Decrease Rate (%/day) | SEC Purity (Aggregates) Daily Increase Rate (%/day) | RP-HPLC Purity (Total Related Substances) Daily Increase Rate (%/day) |
|---|---|---|---|---|---|
| A16 | Polysorbate 80 | 0.03 | 0.04 | 0.03 | 0.04 |
| A17 | Polysorbate 20 | 0.01 | 0.48 | 0.02 | 0.48 |
| A18 | Poloxamer 188 | 0.02 | 0.85 | 0.01 | 0.85 |
| A19 | Poloxamer 407 | 0.02 | 1.70 | 0.02 | 1.70 |

From Table 18 and Table 19, it can be concluded that different types of surfactants have a relatively small impact on SEC purity and aggregate formation but a significant impact on RP-HPLC purity. Based on the analysis of the daily decrease rate of RP-HPLC purity (main peak + peak 4) and the daily increase rate of RP-HPLC purity (total related substances), Polysorbate 80 or Polysorbate 20 as stabilizers show superior performance. However, when Poloxamer 188 is used as a stabilizer, the formulation stability remains within acceptable standards.

3.2 Preparation of high-concentration injection formulations F12 and F13 with different surfactants. The final concentration of the GLP-1-Fc-FGF21 dual-target fusion protein (referred to as the fusion protein or API) and the types and final concentrations of excipients in the injections are detailed in Table 20. The preparation method for the injections is as follows: the excipients and API were weighed and mixed according to the formulation table. If necessary, the pH of the solution was adjusted to the desired value using a 2% hydrochloric acid solution or a 2% sodium hydroxide solution. Finally, the mixture was diluted with water for injection to a total volume of 15 ml. After preparation, the solution was filtered through 0.45 µm and 0.22 µm PVDF membranes in series into 1 ml long thin pre-filled syringes, with a fill volume of 0.5 ml per syringe, and then sealed with rubber stoppers. Formulations F12 and F13 were subjected to high-temperature stability studies. The rate of change in SEC-HPLC aggregates (day/%) after storage at 40°C for 28 days is shown in Table 21, and the appearance inspection results are shown in Table 22.

**Table 20: Ratios of Components in Formulations F12 and F13**

| Formulation | Surfactant Type | Surfactant Concentration (mg/ml) | GLP-1-Fc-FGF21 Dual-Target Fusion Protein Concentration (mg/ml) | Mannitol (mg/ml) | Histidine(mM) | Formulation pH |
|---|---|---|---|---|---|---|
| F12 | Polysorbate 80 | 1.0 | 40 | 44.5 | 10 | 7.1 |
| F13 | Polysorbate 20 | 0.2 | 40 | 44.5 | 10 | 7.1 |

**Table 21: Rate of Change in SEC-HPLC Aggregates for Formulations F12~F13 After Storage at 40°C for 28 Days**

| Formulation | 0 Days - SEC Aggregates (%) | SEC Aggregates at 40°C for 28 Days (%) | Rate of Change (%/day) |
|---|---|---|---|
| F12 | 1.49 | 2.76 | 0.05 |
| F13 | 1.50 | 2.69 | 0.04 |

**Table 22: Appearance Inspection Results for Formulations F12 and F13 After Storage at 40°C for 28 Days**

| Formulation | 0 Day- Appearance | Appearance at 40°C for 28 Days |
|---|---|---|
| F12 | Clear liquid with slight opalescence | Clear liquid with slight opalescence |
| F13 | Clear liquid with slight opalescence | Clear liquid with slight opalescence |

The data from Table 21 and Table 22 demonstrate that formulations prepared with Polysorbate 80 and Polysorbate 20 effectively slow down the growth of SEC aggregates and prevent the formation of visible particles, provide excellent stabilizing effects on the fusion protein.

### 4. Evaluation of Injections Prepared with Different Osmotic Pressure Regulators

4.1 Injections of low concentration injections A23~A25 were prepared using different osmotic pressure regulators. The final concentration of the GLP-1-Fc-FGF21 dual-target fusion protein (referred to as the fusion protein) and the types and final concentrations of excipients in the injections are detailed in Table 23. The preparation method for the injections is described in Step 2.1 of the experimental procedure in this application. Formulations A23~A25 were subjected to high-temperature stability studies. The SEC-HPLC stability test results after storage at 40°C for 0 days, 7 days, and 14 days are shown in Table 24.

**Table 23: Ratios of Components in Formulations A23~A25**

| Formulation | Concentration of GLP-1-Fc-FGF21 Dual-Target Fusion Protein (mg/ml) | Buffer System and Its Amount (10 mM) | Polysorbate 80 Concentration (mg/ml) | Osmotic Pressure Regulator and Concentration | pH Value |
|---|---|---|---|---|---|
| A23 | 2 | Citric Acid Monohydrate (0.14 mg/ml), Sodium Citrate Dihydrate (2.74 mg/ml) | 0.2 | Mannitol (46.4mg/ml) | 6.5 |
| A24 | 2 | Citric Acid Monohydrate (0.14 mg/ml), Sodium Citrate Dihydrate (2.74 mg/ml) | 0.2 | Sodium chloride (8.77mg/ml) | 6.5 |
| A25 | 2 | Citric Acid Monohydrate (0.14 mg/ml), Sodium Citrate Dihydrate (2.74 mg/ml) | 0.2 | Sodium chloride (5.84mg/ml) , sucrose (10mg/ml) | 6.5 |

**Table 24: Stability Results for Formulations A23~A25 Under High-Temperature Conditions (40°C)**

| Test Item | Osmotic Pressure Regulator Type | Formulation | High Temperature 40°C | | |
|---|---|---|---|---|---|
| | | | 0 days | 10 days | 14 days |
| SEC-Purity (Monomer Including Shoulder Peaks) (%) | Mannitol | A23 | 96.91 | 97.42 | 96.92 |
| | Sodium chloride | A24 | 97.89 | 97.02 | 96.77 |
| | Sodium chloride, sucrose | A25 | 97.88 | 96.86 | 96.62 |
| SEC-Purity (Aggregates) (%) | Mannitol | A23 | 2.21 | 1.00 | 1.27 |
| | Sodium chloride | A24 | 1.32 | 1.20 | 1.13 |
| | Sodium chloride, sucrose | A25 | 1.31 | 1.32 | 1.25 |
| RP-HPLC-Purity (Main Peak + Peak 4) (%) | Mannitol | A23 | 72.17 | 60.74 | 56.30 |
| | Sodium chloride | A24 | 73.28 | 59.25 | 53.59 |
| | Sodium chloride, sucrose | A25 | 74.32 | 59.39 | 53.03 |
| RP-HPLC-Purity (Total Related Substances) (%) | Mannitol | A23 | 27.83 | 39.26 | 43.70 |
| | Sodium chloride | A24 | 26.72 | 40.75 | 46.41 |
| | Sodium chloride, sucrose | A25 | 25.68 | 40.61 | 46.97 |
| Visible Particles* | Mannitol | A23 | Complies with Specificati ons | Complies with Specificati ons | Complies with Specificatio ns |
| | Sodium chloride | A24 | Complies with Specificati ons | Complies with Specificati ons | Does Not Comply with Specificatio ns |
| | Sodium chloride, sucrose | A25 | Complies with Specificati ons | Complies with Specificati ons | Does Not Comply with Specificatio ns |

From Table 24, it can be observed that compared to formulations using sodium chloride or a combination of sodium chloride and sucrose, formulations using mannitol as the osmotic pressure regulator exhibit the slowest growth rate in RP-HPLC purity (total related substances) and fewer visible particles. Therefore, formulations prepared with mannitol as the osmotic pressure regulator demonstrate superior stability.

Additionally, the osmotic pressure values of low-concentration injections prepared with mannitol were measured and found to be equal to or close to human plasma osmotic pressure. The results are as follows:
Formulation A12: 307 mOsm/kg,
Formulation A16: 310 mOsm/kg,
Formulation A26: 309 mOsm/kg,
Formulation A29: 325 mOsm/kg.

4.2 Preparation of low-concentration injection formulations A34~A35 with different osmotic pressure regulators. The final concentration of the GLP-1-Fc-FGF21 dual-target fusion protein (referred to as the fusion protein) and the types and final concentrations of excipients in the injections are detailed in Table 25. The preparation method for the injections is described in Step 2.1 of the experimental procedure in this application. Formulations A34~A35 were subjected to high-temperature stability studies. The SEC-HPLC stability test results after storage at 40°C for 0 days, 7 days, and 14 days are shown in Table 26.

**Table 24: Ratios of Components in Formulations A34~A35**

| Formulation | Concentration of GLP-1-Fc-FGF21 Dual-Target Fusion Protein (mg/ml) | Buffer System and Its Amount (10 mM) | Polysorbate 80 Concentration (mg/ml) | Osmotic pressure regulator and Concentration | pH Value |
|---|---|---|---|---|---|
| A34 | 3 | Citric Acid Monohydrate (0.14 mg/ml), Sodium Citrate Dihydrate (2.74 mg/ml) | 0.2 | Sucrose (90mg/ml) | 6.5 |
| A35 | 3 | Citric Acid Monohydrate (0.14 mg/ml), Sodium Citrate Dihydrate (2.74 mg/ml) | 0.2 | Trehalose (60mg/ml) | 6.5 |

**Table 25: Stability Results for Formulations A34~A35 Under High-Temperature Conditions (40°C)**

| Test Item | Osmotic pressure regulator type | Formulation | High Temperature 40°C | | |
|---|---|---|---|---|---|
| | | | 0 days | 10 days | 14 days |
| SEC-Purity (Monomer Including Shoulder Peaks) (%) | Sucrose | A34 | 99.248 | 99.054 | 98.170 |
| | Trehalose | A35 | 99.092 | 98.788 | 98.310 |
| SEC-Purity (Aggregates) (%) | Sucrose | A34 | 0.753 | 0.946 | 1.480 |
| | Trehalose | A35 | 0.907 | 1.117 | 1.680 |
| RP-HPLC-Purity (Main Peak + Peak 4) (%) | Sucrose | A34 | 73.88 | 69.62 | 71.21 |
| | Trehalose | A35 | 78.20 | 68.96 | 64.51 |
| RP-HPLC-Purity (Total Related Substances) (%) | Sucrose | A34 | 26.12 | 30.38 | 28.79 |
| | Trehalose | A35 | 21.80 | 31.04 | 35.49 |
| Visible particles | Sucrose | A34 | Complies with Specificati ons | Complies with Specificati ons | Complies with Specificatio ns |
| | Trehalose | A35 | Complies with Specificati ons | Complies with Specificati ons | Complies with Specificatio ns |

Formulations prepared using sucrose or trehalose as osmotic pressure regulators also demonstrate excellent stability.

4.3 Preparation of High-Concentration Injection Formulations F14 and F15 with Different Osmotic Pressure Regulators. The final concentration of the GLP-1-Fc-FGF21 dual-target fusion protein (referred to as the fusion protein or API) and the types and final concentrations of excipients in the injections are detailed in Table 26. The preparation method for the injections is as follows: the excipients and API were weighed and mixed according to the formulation table. If necessary, the pH of the solution was adjusted to the desired value using a 2% hydrochloric acid solution or a 2% sodium hydroxide solution. Finally, the mixture was diluted with water for injection to a total volume of 9 ml. After preparation, the solution was filtered through 0.45 µm and 0.22 µm PVDF membranes in series into 1 ml long thin pre-filled syringes, with a fill volume of 0.5 ml per syringe, and then sealed with rubber stoppers. Formulations F14 and F15 were subjected to high-temperature stability studies. The rate of change in SEC-HPLC aggregates (day/%) after storage at 40°C for 30 days is shown in Table 27, and the appearance inspection results after storage at 40°C for 14 days are shown in Table 28.

**Table 26: Ratios of Components in Formulations F14 and F15**

| Formulation | Osmotic Pressure Regulator Type | Osmotic Pressure Regulator Concentration (mg/ml) | GLP-1-Fc-FGF21 Dual-Target Fusion Protein Concentration (mg/ml) | Polysorbate 80 Concentration (mg/ml) | Histidine (mM) | Formulation pH |
|---|---|---|---|---|---|---|
| F14 | Mannitol | 40 | 40 | 0.5 | 10 | 7.0 |
| F15 | Sucrose | 90 | 40 | 0.5 | 10 | 7.0 |

**Table 27: Rate of Change in SEC-HPLC Aggregates for Formulations F14 and F15 After Storage at 40°C for 30 Days**

| Formulation | 0 Days - SEC Aggregates (%) | 30 Days at 40°C - SEC Aggregates (%) | Rate of Change (%/day) |
|---|---|---|---|
| F14 | 1.99 | 3.43 | 0.05 |
| F15 | 2.03 | 3.36 | 0.04 |

**Table 28: Appearance Inspection Results for Formulations F14 and F15 After Storage at 40°C for 14 Days**

| Formulation | 0-Day Appearance | Appearance at 40°C for 14 Days |
|---|---|---|
| F14 | Clear liquid with slight opalescence | Clear liquid with slight opalescence |
| F15 | Clear liquid with slight opalescence | Clear liquid with slight opalescence |

To further evaluate the impact of different osmotic pressure regulators on the conformational stability and colloidal stability of the protein, the Prometheus NT.48 protein stability analyzer from NanoTemper was used to test formulations F14~F15 using the ThermControl module. This analysis provided the protein's denaturation temperature (Tm and Tonset) and the onset temperature for protein aggregation (Tagg). The test results are shown in Table 29 below.

**Table 29: Tagg and Tm Values for Formulations F14~F15**

| Formulation | Tagg (°C) | Tonset (°C) | Tm1 (°C) | Tm2 (°C) |
|---|---|---|---|---|
| F14 | No Aggregation Detected | 56.32 | 63.42 | 80.34 |
| F15 | No Aggregation Detected | 56.77 | 64.16 | 80.89 |

From Tables 27~29, it can be concluded that both mannitol and sucrose enable the dual-target fusion protein formulations to maintain good colloidal stability and conformational stability. They also slow down the rate of protein aggregation and prevent the formation of visible particles, providing excellent stabilizing effects on the fusion protein.

### 5. Evaluation of Injections Prepared with Different Histidine Concentrations

Preparation of High-Concentration Injection Formulations F16~F18 with Different Histidine Concentrations. The final concentration of the GLP-1-Fc-FGF21 dual-target fusion protein (referred to as the fusion protein or API) and the types and final concentrations of excipients in the injections are detailed in Table 30. The preparation method for the injections is as follows: the excipients and API were weighed and mixed according to the formulation table. If necessary, the pH of the solution was adjusted to the desired value using a 2% hydrochloric acid solution or a 2% sodium hydroxide solution. Finally, the mixture was diluted with water for injection to a total volume of 10 ml. After preparation, the solution was filtered through 0.45 µm and 0.22 µm PVDF membranes in series into 1 ml long thin pre-filled syringes, with a fill volume of 0.5 ml per syringe, and then sealed with rubber stoppers. Formulations F16~F18 were subjected to high-temperature stability studies. The rate of change in SEC-HPLC aggregates (day/%) after storage at 40°C for 30 days is shown in Table 31.

**Table 30: Ratios of Components in Formulations F16~F18**

| Formulation | Histidine hydrochloride Concentration (mM) | Mannitol Concentration (mg/ml) | GLP-1-Fc-FGF21 Dual-Target Fusion Protein Concentration (mg/ml) | Polysorbate 80 Concentration (mg/ml) | Formulation pH |
|---|---|---|---|---|---|
| F16 | 10 | 46.4 | 40 | 0.5 | 7.0 |
| F17 | 15 | 46.4 | 40 | 0.5 | 7.0 |
| F18 | 20 | 46.4 | 40 | 0.5 | 7.0 |

**Table 31: Rate of Change in SEC-HPLC Aggregates for Formulations F16~F18 After Storage at 40°C for 30 Days**

| Formulation | 0 Days - SEC Aggregates (%) | 30 Days at 40°C - SEC Aggregates (%) | Rate of Change (%/day) |
|---|---|---|---|
| F16 | 0.89 | 2.76 | 0.09 |
| F17 | 1.00 | 2.97 | 0.10 |
| F18 | 0.99 | 3.14 | 0.11 |

From the above, it can be seen that when the histidine concentration is 10-20 mM, the growth rate of SEC aggregates in the fusion protein is relatively slow, and it has a good stabilizing effect on the fusion protein.

### 6. Investigation of Injections Prepared with Different Polysorbate 80 Concentration

6.1 Injections with low concentrations (A20-A22 and A32-A33) were prepared using different concentrations of polysorbate 80. The final concentration of the GLP-1-Fc-FGF21 dual-target fusion protein (referred to as the fusion protein) and the types and final concentrations of excipients in the injections are detailed in Table 32. The preparation method for the injections is described in step 2.1 of the examples in this application. The formulations A20-A22 and A32-A33 were then subjected to high-temperature stability studies. The SEC-HPLC stability test results after storage at 40°C for 0 days, 7 days, 14 days, and 28 days are shown in Table 33.

**Table 32: Ratios of the Components in Formulations A20-A22 and A32-A33.**

| Formulation | Concentration of GLP-1-Fc-FGF21 Dual-Target Fusion Protein (mg/ml) | Buffer System and Its Amount (10 mM) | Polysorbate 80 Concentration (mg/ml) | Mannitol (mg/ml) | pH Value |
|---|---|---|---|---|---|
| A20 | 3 | Citric Acid Monohydrate (0.14 mg/ml), Sodium Citrate Dihydrate (2.74 mg/ml) | 0.1 | 46.4 | 6.5 |
| A21 | 3 | Citric Acid Monohydrate (0.14 mg/ml), Sodium Citrate Dihydrate (2.74 mg/ml) | 0.2 | 46.4 | 6.5 |
| A22 | 3 | Citric Acid Monohydrate (0.14 mg/ml), Sodium Citrate Dihydrate (2.74 mg/ml) | 0.3 | 46.4 | 6.5 |
| A32 | 3 | Citric Acid Monohydrate (0.14 mg/ml), Sodium Citrate Dihydrate (2.74 mg/ml) | 0.4 | 46.4 | 6.5 |
| A33 | 3 | Citric Acid Monohydrate (0.14 mg/ml), Sodium Citrate Dihydrate (2.74 mg/ml) | 0.5 | 46.4 | 6.5 |

**Table 33: Stability Results for Formulations A20~A22 and A32~A33 Under High-Temperature Conditions (40°C)**

| Test Item | Formulation | High Temperature 40°C | | | |
|---|---|---|---|---|---|
| | | 0 days | 7 days | 14 days | 28 days |
| SEC-Purity (Monomer Including Shoulder Peaks) (%) | A20 | 98.24 | 98.24 | 97.57 | 96.53 |
| | A21 | 98.48 | 98.60 | 97.70 | 96.15 |
| | A22 | 98.46 | 98.69 | 97.65 | 96.10 |
| | A32 | 98.44 | 98.30 | 97.64 | 95.98 |
| | A33 | 98.42 | 98.41 | 97.37 | 95.73 |
| SEC-Purity (Aggregates) (%) | A20 | 1.41 | 1.40 | 1.46 | 1.35 |
| | A21 | 1.46 | 1.04 | 1.28 | 1.55 |
| | A22 | 1.50 | 0.98 | 1.33 | 1.62 |
| | A32 | 1.51 | 1.32 | 1.36 | 1.73 |
| | A33 | 1.52 | 1.23 | 1.62 | 1.94 |
| RP-HPLC-Purity (Main Peak + Peak 4) (%) | A20 | 77.68 | 67.90 | 61.64 | 48.91 |
| | A21 | 76.31 | 68.34 | 60.12 | 47.28 |
| | A22 | 76.24 | 63.41 | 61.57 | 46.91 |
| | A32 | 75.83 | 63.80 | 60.77 | 48.06 |
| | A33 | 76.07 | 66.50 | 58.71 | 46.88 |
| RP-HPLC-Purity (Total Related Substances) (%) | A20 | 22.32 | 32.10 | 38.36 | 51.09 |
| | A21 | 23.69 | 31.66 | 39.88 | 52.72 |
| | A22 | 23.76 | 36.59 | 38.43 | 53.09 |
| | A32 | 24.17 | 36.20 | 39.23 | 51.94 |
| | A33 | 23.93 | 33.50 | 41.29 | 53.12 |
| Insoluble Particle (≥10µm) | A20 | 25 | / | / | 69 |
| | A21 | 21 | / | / | 16 |
| | A22 | 11 | / | / | 30 |
| | A32 | 14 | / | / | 39 |
| | A33 | 11 | / | / | 83 |
| Insoluble Particle (≥25µm) | A20 | 2 | / | / | 2 |
| | A21 | 5 | / | / | 2 |
| | A22 | 9 | / | / | 0 |
| | A32 | 0 | / | / | 5 |
| | A33 | 2 | / | / | 14 |
| Visible particles* | A20 | Complies with Specifications | Complies with Specifications | Complies with Specifications | Complies with Specifications |
| | A21 | Complies with Specifications | Complies with Specifications | Complies with Specifications | Complies with Specifications |
| | A22 | Complies with Specifications | Complies with Specifications | Complies with Specifications | Complies with Specifications |
| | A32 | Complies with Specifications | Complies with Specifications | Complies with Specifications | Complies with Specifications |
| | A33 | Complies with Specifications | Complies with Specifications | Complies with Specifications | Complies with Specifications |

| | | | | | |
|---|---|---|---|---|---|
| Note: "/" indicates not detected. | | | | | |

From Table 33, it can be seen that when the dosage of polysorbate 80 is within the range of 0.1 mg/ml to 0.5 mg/ml, the stability of the injections is optimal.

6.2 High-concentration injection formulations F19-F25 were prepared using different concentrations of polysorbate 80. The final concentration of the GLP-1-Fc-FGF21 dual-target fusion protein (referred to as the fusion protein, i.e., API) and the types and final concentrations of excipients in the injections are detailed in Table 34. The preparation method for the injections is as follows: the excipients and API were weighed and mixed according to the table below. If necessary, the pH of the solution was adjusted to the corresponding value using a 2% hydrochloric acid solution or a 2% sodium hydroxide solution. Finally, the mixture was diluted with water for injection to a total volume of 15 ml. After preparation, the solution was filtered sequentially through 0.45 µm and 0.22 µm PVDF membranes into 1 ml slender pre-filled syringes, with a filling volume of 0.5 ml per syringe, and then sealed with rubber stoppers. The formulations F19-F25 were then subjected to high-temperature stability studies. The SEC-HPLC aggregate growth rate (%/day) after storage at 40°C for 28 days is shown in Table 35, the appearance inspection results are shown in Table 36, and the insoluble particle test results are shown in Table 37.

**Table 34: Ratios of the components in formulations F19-F25**

| Formulation | Polysorbate 80 Concentration (mM) | Mannitol Concentration (mg/ml) | GLP-1-Fc-FGF21 Dual-Target Fusion Protein Concentration (mg/ml) | Histidine Concentration (mM) | Formulation pH |
|---|---|---|---|---|---|
| F19 | 0.2 | 44.5 | 40 | 10 | 7.1 |
| F20 | 0.3 | 44.5 | 40 | 10 | 7.1 |
| F21 | 0.4 | 44.5 | 40 | 10 | 7.1 |
| F22 | 0.5 | 44.5 | 40 | 10 | 7.1 |
| F23 | 0.7 | 44.5 | 50 | 10 | 7.1 |
| F24 | 1.0 | 44.5 | 50 | 10 | 7.1 |
| F25 | 1.3 | 44.5 | 50 | 10 | 7.1 |

**Table 35: SEC-HPLC Aggregate Growth Rate Results for Formulations F19-F25 After Storage at 40°C for 28 Days**

| Formulation | 0 Days - SEC Aggregates (%) | 28 Days at 40°C - SEC Aggregates (%) | Rate of Change (%/day) |
|---|---|---|---|
| F19 | 1.19 | 2.31 | 0.04 |
| F20 | 1.21 | 2.46 | 0.04 |
| F21 | 1.21 | 2.34 | 0.04 |
| F22 | 1.21 | 2.57 | 0.05 |
| F23 | 1.33 | 3.82 | 0.09 |
| F24 | 1.32 | 3.76 | 0.09 |
| F25 | 1.33 | 3.86 | 0.09 |

**Table 36: Appearance Inspection Results for Formulations F19~F25 After Storage at 40°C for 28 Days**

| Formulation | 0 Days-Appearance | 28 Days at 40°C-Appearance |
|---|---|---|
| F19 | Clear liquid with slight opalescence | A small number of white particles were observed |
| F20 | Clear liquid with slight opalescence | A small number of white particles were observed |
| F21 | Clear liquid with slight opalescence | Clear liquid with slight opalescence |
| F22 | Clear liquid with slight opalescence | Clear liquid with slight opalescence |
| F23 | Clear liquid with slight opalescence | Clear liquid with slight opalescence |
| F24 | Clear liquid with slight opalescence | Clear liquid with slight opalescence |
| F25 | Clear liquid with slight opalescence | Clear liquid with slight opalescence |

**Table 37: Detection results of insoluble particles for formulations F19~F25 after storage at high temperature (40°C) for 28 days.**

| Formulation | Insoluble particles ≥10µm (particles/ml) | | Insoluble particles ≥25µm (particles/ml) | |
|---|---|---|---|---|
| | 0 days | High temperature (40°C) for 28 days | 0 days | High temperature (40°C) for 28 days |
| F19 | 3 | 232 | 0 | 0 |
| F20 | 3 | 196 | 0 | 3 |
| F21 | 5 | 7 | 3 | 3 |
| F22 | 5 | 3 | 0 | 3 |
| F23 | 12 | 3 | 0 | 0 |
| F24 | 3 | 10 | 0 | 3 |
| F25 | 0 | 0 | 0 | 0 |

From the above, it can be concluded that when the concentration of polysorbate 80 is in the range of 0.4~1.3 mg/ml, it can maintain the stability of the dual-target fusion protein. The growth rate of protein aggregates is relatively slow, the number of insoluble particles is low, and it prevents the formation of visible particles as well as reduces the formation of insoluble particles.

### 7. Investigation of Injections Prepared with Different Fusion Protein Concentrations

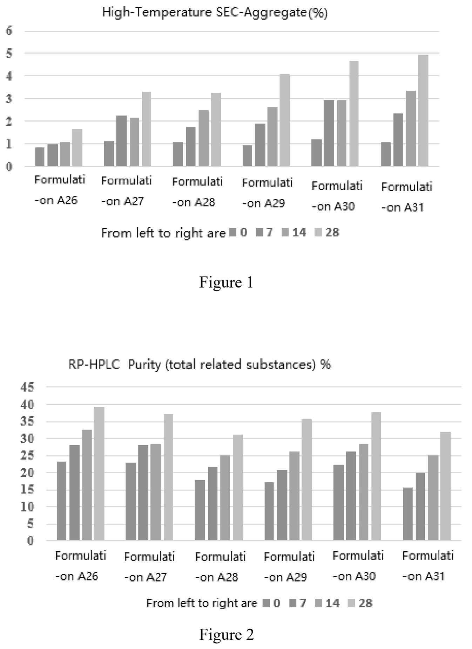

7.1 Preparation of Low-Concentration Injection Formulations A26~A31 Using Different Fusion Protein Concentrations. The final concentration of the GLP-1-Fc-FGF21 dual-target fusion protein (referred to as the fusion protein) and the types and final concentrations of excipients in the injections are detailed in Table 38. The preparation method for the injections is described in Step 2.1 of the embodiments of this application. Subsequently, formulations A26~A31 were subjected to high-temperature stability studies. The SEC-HPLC stability test results after storage at 40°C for 0, 7, 14, and 28 days are shown in Table 39. The growth trends of aggregates for different concentration formulations under high-temperature SEC conditions are illustrated in Figure 1, and the growth trends of purity (total related substances) under high-temperature RP-HPLC conditions are shown in Figure 2.

**Table 38: Ratios of components in formulations A26~A31.**

| Formulation | Concentration of GLP-1-Fc-FGF21 Dual-Target Fusion Protein (mg/ml) | Buffer System and Its Amount (10 mM) | Polysorbate 80 Concentration (mg/ml) | Mannitol Concentration (mg/ml) | pH Value |
|---|---|---|---|---|---|
| A26 | 3 | Citric Acid Monohydrate (0.14 mg/ml), Sodium Citrate Dihydrate (2.74 mg/ml) | 0.2 | 46.4 | 6.5 |
| A27 | 10 | Citric Acid Monohydrate (0.14 mg/ml), Sodium Citrate Dihydrate (2.74 mg/ml) | 0.2 | 46.4 | 6.5 |
| A28 | 15 | Citric Acid Monohydrate (0.14 mg/ml), Sodium Citrate Dihydrate (2.74 mg/ml) | 0.2 | 46.4 | 6.5 |
| A29 | 17 | Citric Acid Monohydrate (0.14 mg/ml), Sodium Citrate Dihydrate (2.74 mg/ml) | 0.2 | 46.4 | 6.5 |
| A30 | 20 | Citric Acid Monohydrate (0.14 mg/ml), Sodium Citrate Dihydrate (2.74 mg/ml) | 0.2 | 46.4 | 6.5 |
| A31 | 25 | Citric Acid Monohydrate (0.14 mg/ml), Sodium Citrate Dihydrate (2.74 mg/ml) | 0.2 | 46.4 | 6.5 |

**Table 39: Stability Results of Formulations A26~A31 under High-Temperature Conditions (40°C)**

| Test Item | Formula tion | High Temperature 40°C | | | |
|---|---|---|---|---|---|
| | | 0 days | 7 days | 14 days | 28 days |
| SEC-Purity (Monomer Including Shoulder Peaks) (%) | A26 | 99.18 | 98.95 | 98.81 | 97.83 |
| | A27 | 98.86 | 97.70 | 97.73 | 96.22 |
| | A28 | 98.94 | 98.18 | 97.26 | 96.43 |
| | A29 | 99.07 | 97.99 | 97.18 | 95.31 |
| | A30 | 98.76 | 97.01 | 97.03 | 94.78 |
| | A31 | 98.94 | 97.56 | 96.47 | 94.60 |
| SEC-Purity (Aggregates) (%) | A26 | 0.82 | 0.99 | 1.06 | 1.68 |
| | A27 | 1.12 | 2.26 | 2.17 | 3.30 |
| | A28 | 1.06 | 1.74 | 2.48 | 3.24 |
| | A29 | 0.93 | 1.90 | 2.64 | 4.10 |
| | A30 | 1.22 | 2.95 | 2.92 | 4.66 |
| | A31 | 1.06 | 2.33 | 3.33 | 4.97 |
| RP-HPLC-Purit y (Main Peak + Peak 4) (%) | A26 | 76.91 | 72.01 | 67.54 | 60.86 |
| | A27 | 77.18 | 72.10 | 71.80 | 62.89 |
| | A28 | 82.21 | 78.33 | 75.01 | 68.99 |
| | A29 | 82.97 | 79.29 | 73.80 | 64.38 |
| | A30 | 77.71 | 73.65 | 71.78 | 62.13 |
| | A31 | 84.36 | 80.26 | 74.89 | 68.01 |
| RP-HPLC-Purit y (Total Related Substances) (%) | A26 | 23.09 | 27.99 | 32.46 | 39.14 |
| | A27 | 22.82 | 27.90 | 28.21 | 37.11 |
| | A28 | 17.80 | 21.67 | 24.99 | 31.01 |
| | A29 | 17.03 | 20.71 | 26.20 | 35.62 |
| | A30 | 22.29 | 26.35 | 28.22 | 37.87 |
| | A31 | 15.64 | 19.74 | 25.11 | 31.99 |
| Visible Particles | A26 | Complies with Specifications | Complies with Specifications | Complies with Specifications | Complies with Specification s |
| | A27 | Complies with Specifications | Complies with Specifications | Complies with Specifications | Does Not Comply with Specification s |
| | A28 | Complies with Specifications | Complies with Specifications | Complies with Specifications | Complies with Specification s |
| | A29 | Complies with Specifications | Complies with Specifications | Complies with Specifications | Complies with Specification s |
| | A30 | Complies with Specifications | Complies with Specifications | Complies with Specifications | Does Not Comply with Specification s |
| | A31 | Complies with Specifications | Complies with Specifications | Complies with Specifications | Complies with Specification s |

From Table 39 and Figures 1-2, it can be observed that the growth rate of RP-HPLC purity (total related substances) does not show significant differences among formulations with different concentrations. However, the growth rate of SEC purity (aggregates) (%) increases with higher formulation concentrations.

Furthermore, the long-term stability of formulations A29 (17 mg/ml) and A30 (20 mg/ml) at 2-8°C was investigated. The test results are shown in Table 40.

**Table 40: Stability Results of Formulations A29~A30 under 2-8°C Conditions**

| Test Item | Formula tion | Long-Term Storage at 2-8°C | | | | | | | |
|---|---|---|---|---|---|---|---|---|---|
| | | 0 month s | 2 month s | 4.5 month s | 5 month s | 6 month s | 8 month s | 11 month s | 12 month s |
| SEC-Purity (Monomer Including Shoulder Peaks) (%) | A29 | 99.00 | 98.52 | / | 98.40 | 98.37 | / | / | / |
| | A30 | 98.76 | / | 98.03 | / | / | 97.25 | 97.46 | 97.32 |
| SEC-Purity (Aggregate s) (%) | A29 | 1.00 | 1.39 | / | 1.60 | 1.63 | / | / | / |
| | A30 | 1.22 | / | 1.94 | / | / | 2.69 | 2.48 | 2.61 |
| RP-HPLC-Purity (Main Peak + Peak 4) (%) | A29 | 81.82 | 81.53 | / | 81.85 | 81.33 | / | / | / |
| | A30 | 77.71 | / | 78.34 | / | / | 75.55 | 76.79 | 73.95 |
| RP-HPLC-Purity (Total Related Substances ) (%) | A29 | 18.18 | 18.47 | / | 18.15 | 18.67 | / | / | / |
| | A30 | 22.29 | / | 21.66 | / | / | 24.45 | 23.21 | 26.05 |
| Visible Particles | A29 | / | Compl ies with Specif ication s | Compl ies with Specif ication s | Compl ies with Specif ication s | Compl ies with Specif ication s | / | / | / |
| | A30 | Compl ies with Specif ication s | / | Compl ies with Specif ication s | / | / | Compl ies with Specif ication s | Compl ies with Specif ication s | / |

| | | | | | | | | | |
|---|---|---|---|---|---|---|---|---|---|
| Note: "/" indicates not tested. | | | | | | | | | |

From Table 40, it can be observed that over the storage period, the RP-HPLC purity (main peak + peak 4) of formulations with different concentrations did not show significant changes. The SEC purity (aggregates) exhibited a slight increasing trend but remained within the acceptable standard range (≤5%).

7.2 Preparation of Low-Concentration Injection Formulations F26~F29 Using Different Fusion Protein Concentrations. The final concentration of the GLP-1-Fc-FGF21 dual-target fusion protein (referred to as the fusion protein) and the types and final concentrations of excipients in the injections are detailed in Table 41. The preparation method for the injections is described in Step 2.1 of the embodiments of this application. Subsequently, formulations F26~F29 were subjected to high-temperature stability studies. The SEC-HPLC stability test results after storage at 40°C for 0, 7, 14, and 28 days showed that the SEC purity (aggregates) of formulations with different concentrations exhibited a slight increasing trend but remained within the acceptable standard range (≤5%). This embodiment exemplarily presents the results of formulation F26, as detailed in Table 42 and Table 43.

**Table 41: Ratios of Components in Formulations F26~F29.**

| Formulation | Concentration of GLP-1-Fc-FGF21 Dual-Target Fusion Protein (mg/ml) | Buffer System and Its Amount | Polysorbate 80 Concentration (mg/ml) | Mannitol Concentration (mg/ml) | pH Value |
|---|---|---|---|---|---|
| F26 | 60 | 10mM Histidine | 1.0 | 44.5 | 7.1 |
| F27 | 70 | 10mM Histidine | 1.0 | 44.5 | 7.1 |
| F28 | 80 | 10mM Histidine | 1.3 | 40 | 7.1 |
| F29 | 90 | 10mM Histidine | 1.3 | 40 | 7.1 |

**Table 42: Rate of Change in SEC-HPLC Aggregates and RP-HPLC Main Peak Purity Under High Temperature Conditions at 40°C for 28 Days**

| Formulation | 0 Days - SEC Aggregates (%) | 28 Days at 40°C - SEC Aggregates (%) | Rate of Change (%/day) |
|---|---|---|---|
| F26 | 0.61 | 2.65 | 0.07 |

| Formulation | 0 days - Main peak purity % (including peak 4) | High temperature 40°C, 28 days - Main peak purity % (including peak 4) | Rate of Change (%/day) |
|---|---|---|---|
| F26 | 84.04 | 68.90 | 0.54 |

**Table 43: Appearance Test Results After Storage at 40°C for 28 Days**

| Formulation | 0-Day Appearance | 40°C, 28-Day Appearance |
|---|---|---|
| F26 | Clear liquid with slight opalescence | Clear liquid with slight opalescence |

### 8, The Effect of EDTA Sodium Salt on Formulation Stability in Different Buffer Systems

8.1 Preparation of low-concentration injection formulations A36~A37 containing EDTA in a citrate buffer system. The final concentration of the GLP-1-Fc-FGF21 dual-target fusion protein (referred to as the fusion protein) in the injection and the types and final concentrations of excipients are detailed in Table 44. The preparation method for the injection is described in Step 2.1 of the embodiments of this application. Subsequently, the high-temperature stability of formulations A36~A37 was studied. The SEC-HPLC stability test results after storage at 40°C for 0 days, 10 days, 14 days, and 28 days are shown in Table 45.

**Table 44: Composition Ratio of Formulations A36~A37**

| Formulation | Concentration of GLP-1-Fc-FGF21 Dual-Target Fusion Protein (mg/ml) | Buffer System and Its Amount (10 mM) | Polysorbate 80 Concentration (mg/ml) | Mannitol Concentration (mg/ml) | EDTA-2Na (mg/ml) | pH Value |
|---|---|---|---|---|---|---|
| A36 | 3 | Citric Acid Monohydrate (0.14 mg/ml), Sodium Citrate Dihydrate (2.74 mg/ml) | 0.2 | 46.4 | 0 | 6.5 |
| A37 | 3 | Citric Acid Monohydrate (0.14 mg/ml), Sodium Citrate Dihydrate (2.74 mg/ml) | 0.2 | 46.4 | 0.115 | 6.5 |

**Table 45: Stability Results of Formulations A36~A37 Under High Temperature Conditions at 40°C**

| Test Item | Formulat ion | High Temperature 40°C | | | | |
|---|---|---|---|---|---|---|
| | | 0 days | 10 days | 14 days | 28 days | Rate of Change Over 28 Days (%/day) |
| SEC - Main Peak + Shoulder Peak (%) | A36 | 99.168 | 98.869 | 98.110 | 98.038 | 0.04 |
| | A37 | 98.894 | 98.792 | 98.230 | 98.009 | 0.03 |
| SEC-Purity (Aggregates) (%) | A36 | 0.832 | 1.058 | 1.890 | 1.386 | 0.02 |
| | A37 | 1.106 | 1.119 | 1.510 | 1.361 | 0.01 |
| RP-HPLC-P urity (Main Peak + Peak 4) (%) | A36 | 74.31 | 70.58 | 65.83 | 60.85 | 0.48 |
| | A37 | 76.26 | 75.52 | 68.40 | 61.84 | 0.52 |
| RP-HPLC-P urity (Total Related Substances) (%) | A36 | 25.69 | 29.42 | 34.17 | 39.15 | 0.48 |
| | A37 | 23.74 | 24.48 | 31.60 | 38.16 | 0.52 |
| IEX - Main Peak (%) | A36 | 53.778 | 43.358 | 39.699 | 27.818 | 0.927 |
| | A37 | 54.140 | 44.154 | 39.107 | 27.918 | 0.937 |
| IEX - Basic Region (%) | A36 | 5.048 | 7.052 | 7.728 | 9.413 | 0.156 |
| | A37 | 5.016 | 7.018 | 7.915 | 9.603 | 0.164 |
| IEX - Acidic Region (%) | A36 | 41.174 | 49.59 | 52.573 | 62.770 | 0.771 |
| | A37 | 40.843 | 48.826 | 52.978 | 62.476 | 0.773 |
| Visible Particles | A36 | Complies with Specificati ons | Complies with Specificatio ns | Complies with Specification s | Complies with Specificati ons | / |
| | A37 | Complies with Specificati ons | Complies with Specificatio ns | Complies with Specification s | Complies with Specificati ons | / |

8.2 Preparation of High-Concentration Injection Formulations F30~F31 in Histidine System Containing EDTA. The final concentration of the GLP-1-Fc-FGF21 dual-target fusion protein (referred to as the fusion protein) and the types and final concentrations of excipients in the injection are detailed in Table 46. The preparation method for the injection is described in Step 2.1 of the examples in this application. Subsequently, high-temperature stability studies were conducted on formulations F30~F31. The SEC-HPLC stability test results after storage at 40°C for 0 days, 7 days, 14 days, and 30 days are shown in Table 47.

**Table 46: Composition Ratios of Formulations F30~F31**

| Formulation | Concentration of GLP-1-Fc-FGF21 Dual-Target Fusion Protein (mg/ml) | Buffer System and Its Amount | Polysorbate 80 Concentration (mg/ml) | Mannitol Concentration (mg/ml) | EDTA-CaNa2 (mg/ml) | pH Value |
|---|---|---|---|---|---|---|
| F30 | 40 | 10mM Histidine | 0.5 | 40 | 0 | 7 |
| F31 | 40 | 10mM Histidine | 0.5 | 40 | 0.1 | 7 |

**Table 47: Stability Results of Formulations F30~F31 Under High Temperature Conditions at 40°C**

| Test Item | Formulat ion | High Temperature 40°C | | | | |
|---|---|---|---|---|---|---|
| | | 0 days | 10 days | 14 days | 30 days | Rate of Change Over 30 Days (%/day) |
| SEC - Main Peak + Shoulder Peak (%) | F30 | 98.0056 | 97.7071 | 97.1907 | 96.0708 | 0.06 |
| | F31 | 97.9930 | 97.7308 | 97.1723 | 96.1026 | 0.06 |
| SEC-Purity (Aggregates) (%) | F30 | 1.9944 | 2.1931 | 2.6114 | 3.4294 | 0.05 |
| | F31 | 2.0070 | 2.1708 | 2.6211 | 3.4091 | 0.05 |
| RP-HPLC-P urity (Main Peak + Peak 4) (%) | F30 | 77.9993 | 74.6799 | 71.8277 | 62.4104 | 0.52 |
| | F31 | 78.2679 | 74.4605 | 72.8191 | 64.3235 | 0.46 |
| RP-HPLC-P urity (Total Related Substances) (%) | F30 | 22.0007 | 25.3201 | 28.1723 | 37.5896 | 0.52 |
| | F31 | 21.7321 | 25.5395 | 27.1809 | 35.6765 | 0.46 |
| IEX - Main Peak (%) | F30 | 54.9082 | 46.2929 | 39.4633 | 24.3218 | 1.02 |
| | F31 | 55.3597 | 46.8038 | 39.7328 | 23.3132 | 1.07 |
| IEX - Basic Region (%) | F30 | 4.1656 | 7.0918 | 8.8678 | 11.6079 | 0.25 |
| | F31 | 4.1609 | 6.7374 | 8.4669 | 11.1682 | 0.23 |
| IEX - Acidic Region (%) | F30 | 40.9262 | 46.6153 | 51.6690 | 64.0704 | 0.77 |
| | F31 | 40.4793 | 46.4588 | 51.8003 | 65.5185 | 0.83 |
| Visible Particles | F30 | Complies with Specificati ons | Complies with Specificatio ns | Complies with Specification s | Does Not Comply with Specificati ons | / |
| | F31 | Complies with Specificati ons | Complies with Specificatio ns | Complies with Specification s | Does Not Comply with Specificati ons | / |

In summary, whether in a citrate system or a histidine buffer system, the formulation demonstrated excellent stability regardless of whether the EDTA sodium salt was added.

### Example 2: Evaluation of Different Fusion Proteins

The amino acid sequence of the GLP-1-Fc-FGF21 dual-target fusion protein 2 in this example is shown in SEQ ID NO: 2; the amino acid sequence of the GLP-1-Fc-FGF21 dual-target fusion protein 3 in this example is shown in SEQ ID NO: 3; the amino acid sequence of the GLP-1-Fc-FGF21 dual-target fusion protein 4 in this example is shown in SEQ ID NO: 4; the amino acid sequence of the GLP-1-Fc-FGF21 dual-target fusion protein 5 in this example is shown in SEQ ID NO: 5; the amino acid sequence of the GLP-1-Fc-FGF21 dual-target fusion protein 6 in this example is shown in SEQ ID NO: 6; the amino acid sequence of the GLP-1-Fc-FGF21 dual-target fusion protein 7 in this example is shown in SEQ ID NO: 7; the amino acid sequence of the GLP-1-Fc-FGF21 dual-target fusion protein 8 in this example is shown in SEQ ID NO: 8; the amino acid sequence of the GLP-1-Fc-FGF21 dual-target fusion protein 9 in this example is shown in SEQ ID NO: 9; the amino acid sequence of the GLP-1-Fc-FGF21 dual-target fusion protein 10 in this example is shown in SEQ ID NO: 10; the amino acid sequence of the GLP-1-Fc-FGF21 dual-target fusion protein 11 in this example is shown in SEQ ID NO: 11; the amino acid sequence of the GLP-1-Fc-FGF21 dual-target fusion protein 12 in this example is shown in SEQ ID NO: 12. Among them, GLP-1-Fc-FGF21 dual-target fusion proteins 2~11 (referred to as fusion proteins 2~11) were prepared using conventional methods in the field.

The types and final concentrations of the GLP-1-Fc-FGF21 dual-target fusion protein (referred to as the fusion protein) in the injection, as well as the types and final concentrations of excipients, are detailed in Table 48. The preparation method for the injection is described in Step 2.1 of Example 1. Subsequently, stability studies were conducted on formulations A38~A31.

**Table 48: Composition Ratios of Formulations A38~A31**

| Formulation | Types of GLP-1-Fc-FGF21 Dual-Target Fusion Protein | Concentration of Fusion Protein (mg/ml) | Buffer System and Its Amount (10 mM) | Polysorbate 80 Concentration (mg/ml) | Mannitol Concentration (mg/ml) | pH Value |
|---|---|---|---|---|---|---|
| A38 | Fusion Protein 2 | 20 | Citric Acid Monohydrate (0.14 mg/ml), Sodium Citrate Dihydrate (2.74 mg/ml) | 0.2 | 46.4 | 6.5 |
| A39 | Fusion Protein 3 | 20 | Citric Acid Monohydrate (0.14 mg/ml), Sodium Citrate Dihydrate (2.74 mg/ml) | 0.2 | 46.4 | 6.5 |
| A40 | Fusion Protein 4 | 20 | Citric Acid Monohydrate (0.14 mg/ml), Sodium Citrate Dihydrate (2.74 mg/ml) | 0.2 | 46.4 | 6.5 |
| A41 | Fusion Protein 5 | 20 | Citric Acid Monohydrate (0.14 mg/ml), Sodium Citrate Dihydrate (2.74 mg/ml) | 0.2 | 46.4 | 6.5 |
| A42 | Fusion Protein 6 | 20 | Citric Acid Monohydrate (0.14 mg/ml), Sodium Citrate Dihydrate (2.74 mg/ml) | 0.2 | 46.4 | 6.5 |
| A43 | Fusion Protein 7 | 20 | Citric Acid Monohydrate (0.14 mg/ml), Sodium Citrate Dihydrate (2.74 mg/ml) | 0.2 | 46.4 | 6.5 |
| A44 | Fusion Protein 8 | 20 | Citric Acid Monohydrate (0.14 mg/ml), Sodium Citrate Dihydrate (2.74 mg/ml) | 0.2 | 46.4 | 6.5 |
| A45 | Fusion Protein 9 | 20 | Citric Acid Monohydrate (0.14 mg/ml), Sodium Citrate Dihydrate (2.74 mg/ml) | 0.2 | 46.4 | 6.5 |
| A46 | Fusion Protein 10 | 20 | Citric Acid Monohydrate (0.14 mg/ml), Sodium Citrate Dihydrate (2.74 mg/ml) | 0.2 | 46.4 | 6.5 |
| A47 | Fusion Protein 11 | 20 | Citric Acid Monohydrate (0.14 mg/ml), Sodium Citrate Dihydrate (2.74 mg/ml) | 0.2 | 46.4 | 6.5 |
| A48 | Fusion Protein 12 | 20 | Citric Acid Monohydrate (0.14 mg/ml), Sodium Citrate Dihydrate (2.74 mg/ml) | 0.2 | 46.4 | 6.5 |

2. The types and final concentrations of the GLP-1-Fc-FGF21 dual-target fusion protein (referred to as the fusion protein) in the injection, as well as the types and final concentrations of excipients, are detailed in Table 49. The preparation method for the injection is described in Step 2.1 of Example 1. Subsequently, stability studies were conducted on formulations F32~F53.

**Table 49: Composition Ratios of Formulations F32~F53**

| Formulation | Types of GLP-1-Fc-FGF21 Dual-Target Fusion Protein | Concentration of Fusion Protein (mg/ml) | Buffer System and Its Amount (10 mM) | Polysorbate 80 Concentration (mg/ml) | Mannitol Concentration (mg/ml) | pH Value |
|---|---|---|---|---|---|---|
| F32 | Fusion Protein 2 | 40 | 10mM Histidine | 1.0 | 44.5 | 7.1 |
| F33 | Fusion Protein 3 | 40 | 10mM Histidine | 1.0 | 44.5 | 7.1 |
| F34 | Fusion Protein 4 | 40 | 10mM Histidine | 1.0 | 44.5 | 7.1 |
| F35 | Fusion Protein 5 | 40 | 10mM Histidine | 1.0 | 44.5 | 7.1 |
| F36 | Fusion Protein 6 | 40 | 10mM Histidine | 1.0 | 44.5 | 7.1 |
| F37 | Fusion Protein 7 | 40 | 10mM Histidine | 1.0 | 44.5 | 7.1 |
| F38 | Fusion Protein 8 | 40 | 10mM Histidine | 1.0 | 44.5 | 7.1 |
| F39 | Fusion Protein 9 | 40 | 10mM Histidine | 1.0 | 44.5 | 7.1 |
| F40 | Fusion Protein 10 | 40 | 10mM Histidine | 1.0 | 44.5 | 7.1 |
| F41 | Fusion Protein 11 | 40 | 10mM Histidine | 1.0 | 44.5 | 7.1 |
| F42 | Fusion Protein 12 | 40 | 10mM Histidine | 1.0 | 44.5 | 7.1 |
| F43 | Fusion Protein 2 | 80 | 10mM Histidine | 1.0 | 44.5 | 7.1 |
| F44 | Fusion Protein 3 | 80 | 10mM Histidine | 1.0 | 44.5 | 7.1 |
| F45 | Fusion Protein 4 | 80 | 10mM Histidine | 1.0 | 44.5 | 7.1 |
| F46 | Fusion Protein 5 | 80 | 10mM Histidine | 1.0 | 44.5 | 7.1 |
| F47 | Fusion Protein 6 | 80 | 10mM Histidine | 1.0 | 44.5 | 7.1 |
| F48 | Fusion Protein 7 | 80 | 10mM Histidine | 1.0 | 44.5 | 7.1 |
| F49 | Fusion Protein 8 | 80 | 10mM Histidine | 1.0 | 44.5 | 7.1 |
| F50 | Fusion Protein 9 | 80 | 10mM Histidine | 1.0 | 44.5 | 7.1 |
| F51 | Fusion Protein 10 | 80 | 10mM Histidine | 1.0 | 44.5 | 7.1 |
| F52 | Fusion Protein 11 | 80 | 10mM Histidine | 1.0 | 44.5 | 7.1 |
| F53 | Fusion Protein 12 | 80 | 10mM Histidine | 1.0 | 44.5 | 7.1 |

### Example 3: Evaluation of Different Preservative Systems

Low-concentration injection formulations were prepared using different preservative systems. The final concentration of the GLP-1-Fc-FGF21 dual-target fusion protein (referred to as the fusion protein or API) in the injection, as well as the types and final concentrations of excipients, are detailed in Table 50. The preparation method for the injection is as follows: the excipients and API were weighed and mixed according to the formulation amounts listed in Table 1. If necessary, the pH of the formulation was adjusted to the corresponding value using a 2% hydrochloric acid solution or a 2% sodium hydroxide solution. Finally, the solution was adjusted to volume with water for injection. After preparation, the drug solution was filtered through 0.45 µm and 0.22 µm PVDF membranes in series into 1 ml slender pre-filled syringes, with a fill volume of 1 ml per syringe, and then sealed with rubber stoppers. Subsequently, stability studies were conducted on the formulations. The results showed that all formulations exhibited good stability, with the rate of change in SEC-HPLC aggregates (day/%) within acceptable standards, and no visible particles were formed.

**Table 50: Composition Ratios of Each Formulation**

| Formulation | Types of GLP-1-Fc-FGF21 Dual-Target Fusion Protein | Concentration of Fusion Protein (mg/ml) | Buffer System and Its Amount (10 mM) | Polysorbate 80 Concentration (mg/ml) | Mannitol Concentration (mg/ml) | Preservative | pH Value |
|---|---|---|---|---|---|---|---|
| A49 | Fusion Protein 1 | 10 | 10mM Histidine | 1.0 | 46.4mg/ml | Phenol 4 mg/mL | 7.0 |
| A50 | Fusion Protein 1 | 20 | 10mM Histidine | 1.0 | 46.4mg/ml | Phenol 4 mg/mL | 7.0 |
| A51 | Fusion Protein 1 | 10 | 10 mM Citrate | 1.0 | 46.4mg/ml | Phenol 4 mg/mL | 7.0 |
| A52 | Fusion Protein 1 | 20 | 10 mM Citrate | 1.0 | 46.4mg/ml | Phenol 4 mg/mL | 7.0 |
| A53 | Fusion Protein 1 | 20 | 10 mM Citrate | 1.0 | 46.4mg/ml | m-Cresol 3 mg/mL | 7.0 |
| A54 | Fusion Protein 1 | 20 | 10 mM Citrate | 1.0 | 46.4mg/ml | m-Cresol 3 mg/mL | 7.0 |
| A55 | Fusion Protein 1 | 40 | 10mM Histidine | 1.0 | 44.5mg/ml | Phenol 4 mg/mL | 7.0 |
| A56 | Fusion Protein 1 | 60 | 10mM Histidine | 1.0 | 44.5mg/ml | Phenol 4 mg/mL | 7.0 |
| A57 | Fusion Protein 1 | 40 | 10 mM Citrate | 1.0 | 44.5mg/ml | Phenol 4 mg/mL | 7.0 |
| A58 | Fusion Protein 1 | 60 | 10 mM Citrate | 1.0 | 44.5mg/ml | Phenol 4 mg/mL | 7.0 |
| A59 | Fusion Protein 1 | 40 | 10mM Histidine | 1.0 | 44.5mg/ml | m-Cresol 3 mg/mL | 7.0 |
| A60 | Fusion Protein 1 | 60 | 10mM Histidine | 1.0 | 44.5mg/ml | m-Cresol 3 mg/mL | 7.0 |
| A61 | Fusion Protein 1 | 60 | 10mM Histidine | 1.0 | 44.5mg/ml | Phenol 3.5 mg/mL, m-Cresol 0.25~0.35 mg/mL | 7.0 |

In the description of this specification, references to terms such as "one embodiment," "some embodiments," "example," "specific example," or "some examples" mean that the specific features, structures, materials, or characteristics described in connection with the embodiment or example are included in at least one embodiment or example of the present invention. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A composition comprising:
a GLP-1-Fc-FGF21 fusion protein and a buffer.

2. The composition according to claim 1, wherein the GLP-1-Fc-FGF21 fusion protein comprises an amino acid sequence as shown in SEQ ID NO: 1~12;
optionally, the concentration of the GLP-1-Fc-FGF21 fusion protein in the composition is 1~100 mg/mL;
optionally, the concentration of the GLP-1-Fc-FGF21 fusion protein in the composition is 2~80 mg/mL;
optionally, the concentration of the GLP-1-Fc-FGF21 fusion protein in the composition is 5~70 mg/mL;
optionally, the concentration of the GLP-1-Fc-FGF21 fusion protein in the composition is 2~55 mg/mL;
optionally, the concentration of the GLP-1-Fc-FGF21 fusion protein in the composition is 10~65 mg/mL;
optionally, the concentration of the GLP-1-Fc-FGF21 fusion protein in the composition is 20~60 mg/mL;
optionally, the pH value of the composition is 6.2~8.0, preferably 6.4~8.0.

3. The composition according to claim 2, wherein the buffer comprises at least one of a histidine buffer, a citrate buffer, a phosphate buffer, and a Tris buffer;
preferably, the buffer comprises a histidine buffer or a citrate buffer;
optionally, the concentration of the buffer in the composition is 5~30 mM;
optionally, the concentration of the buffer in the composition is 5~25 mM;
optionally, the concentration of the buffer in the composition is 8~25 mM;
optionally, the concentration of the buffer in the composition is 5~20 mM;
optionally, the concentration of the buffer in the composition is 8~12 mM;
preferably, the concentration of the buffer in the composition is 10~20 mM.

4. The composition according to claim 1, wherein the composition further comprises at least one of an osmotic pressure regulator, a surfactant, a preservative, and a metal chelating agent;
optionally, the osmotic pressure regulator comprises at least one of mannitol, sucrose, trehalose, and sodium chloride;
optionally, the concentration of the osmotic pressure regulator in the composition is 30~100 mg/mL or 1~10 mg/mL;
optionally, the surfactant comprises at least one of polysorbate 80, polysorbate 20, and poloxamer 188;
optionally, the concentration of the surfactant in the composition is 0.1~10 mg/mL, preferably 0.1~1.5 mg/mL or 6~10 mg/mL, more preferably 0.1~0.7 mg/mL;
optionally, the preservative comprises at least one of phenol and m-cresol;
optionally, the concentration of the preservative in the composition is 0.1~10 mg/mL, preferably 1.0~5.0 mg/mL;
optionally, the metal chelating agent comprises at least one of disodium edetate and calcium disodium edetate;
optionally, the concentration of the metal chelating agent in the composition is 0.01~1 mg/mL, preferably 0.05~0.5 mg/mL.

5. The composition according to any one of claims 1 to 4, wherein the buffer is a histidine buffer, and the concentration of the GLP-1-Fc-FGF21 fusion protein in the composition is 25~100 mg/mL, preferably 25~95 mg/mL, 25~90 mg/mL, or 25~85 mg/mL;
optionally, the buffer is a histidine buffer, and the concentration of the GLP-1-Fc-FGF21 fusion protein in the composition is 25~80 mg/mL, preferably 25~55 mg/mL;
optionally, the concentration of the histidine buffer in the composition is 5~30 mM, preferably 5~25 mM, 8~25 mM, or 8~20 mM, more preferably 10~20 mM or 8~12 mM;
optionally, the pH of the composition is 6.7~7.5, preferably 6.9~7.5;
optionally, the osmotic pressure regulator is mannitol, and the concentration of the osmotic pressure regulator in the composition is 30~50 mg/mL; or
the osmotic pressure regulator is sucrose, and the concentration of the osmotic pressure regulator in the composition is 60~100 mg/mL, preferably 80~100 mg/mL; or
the osmotic pressure regulator is trehalose, and the concentration of the osmotic pressure regulator in the composition is 40~100 mg/mL, preferably 40~70 mg/mL, more preferably 50~70 mg/mL;
optionally, the surfactant is polysorbate 80, and the concentration of the surfactant in the composition is 0.1~2 mg/mL, preferably 0.1~1.5 mg/mL, more preferably 0.4~1.3 mg/mL; or
the surfactant is polysorbate 20, and the concentration of the surfactant in the composition is 0.1~1.5 mg/mL, preferably 0.1~0.5 mg/mL.

6. The composition according to any one of claims 1 to 4, wherein the concentration of the GLP-1-Fc-FGF21 fusion protein in the composition is 2~25 mg/mL, and the buffer is a citrate buffer;
optionally, the concentration of the citrate buffer in the composition is 5~30 mM, preferably 5~25 mM, 8~25 mM, or 8~20 mM, more preferably 10~20 mM or 8~12 mM;
optionally, the pH of the composition is 6.4~8.0;
optionally, the osmotic pressure regulator is mannitol, and the concentration of the osmotic pressure regulator in the composition is 30~50 mg/mL; or
the osmotic pressure regulator is sucrose, and the concentration of the osmotic pressure regulator in the composition is 60~100 mg/mL, preferably 80~100 mg/mL; or
the osmotic pressure regulator is trehalose, and the concentration of the osmotic pressure regulator in the composition is 40~100 mg/mL, preferably 40~70 mg/mL, more preferably 50~70 mg/mL;
the osmotic pressure regulator is sodium chloride, and the concentration of the osmotic pressure regulator in the composition is 1~10 mg/mL, preferably 3~9 mg/mL;
optionally, the surfactant comprises at least one of polysorbate 80, polysorbate 20, and poloxamer 188, preferably polysorbate 80;
optionally, the concentration of the surfactant in the composition is 0.1~10 mg/mL, preferably 0.1~1.5 mg/mL, 0.1~1.0 mg/mL, or 6~10 mg/mL;
optionally, the surfactant is poloxamer 188, and the concentration of the surfactant in the composition is 6~10 mg/mL; or
the surfactant is polysorbate 80 or polysorbate 20, and the concentration of the surfactant in the composition is 0.1~1.5 mg/mL, preferably 0.1~0.5 mg/mL.

7. The composition according to any one of claims 1 to 4, wherein the dosage form of the composition is a liquid formulation, preferably an injection.

8. An injection comprising:
a GLP-1-Fc-FGF21 fusion protein, a histidine buffer, mannitol, and optionally disodium edetate or calcium disodium edetate, as well as polysorbate 80 or polysorbate 20;
wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL;
the concentration of the histidine buffer in the injection is 8~25 mM, preferably 10~20 mM;
the concentration of mannitol in the injection is 35~45 mg/mL;
the concentration of polysorbate 80 in the injection is 0.4~1.5 mg/mL or 0.4~1.3 mg/mL, or the concentration of polysorbate 20 in the injection is 0.1~0.5 mg/mL;
the concentration of the metal chelating agent in the injection is 0.05~0.5 mg/mL;
the pH of the injection is 6.9~7.5;
preferably, the injection does not contain disodium edetate or calcium disodium edetate.

9. An injection comprising:
a GLP-1-Fc-FGF21 fusion protein,
a histidine buffer,
sucrose or trehalose,
polysorbate 80 or polysorbate 20, and
optionally disodium edetate or calcium disodium edetate;
wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL;
the concentration of the histidine buffer in the injection is 8~25 mM, preferably 10~20 mM;
the concentration of sucrose in the injection is 85~95 mg/mL;
the concentration of trehalose in the injection is 55~65 mg/mL;
the concentration of polysorbate 80 in the injection is 0.4~1.3 mg/mL, or the concentration of polysorbate 20 in the injection is 0.1~0.5 mg/mL;
the pH of the injection is 6.9~7.5;
preferably, the injection does not contain disodium edetate or calcium disodium edetate.

10. An injection comprising:
a GLP-1-Fc-FGF21 fusion protein, a citrate buffer, sucrose or trehalose, and optionally disodium edetate or calcium disodium edetate, as well as polysorbate 80 or polysorbate 20;
wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL;
the concentration of the citrate buffer in the injection is 8~25 mM, preferably 10~20 mM;
the concentration of sucrose in the injection is 85~95 mg/mL;
the concentration of trehalose in the injection is 55~65 mg/mL;
the concentration of polysorbate 80 in the injection is 0.4~1.3 mg/mL, or the concentration of polysorbate 20 in the injection is 0.1~0.5 mg/mL;
the pH of the injection is 6.9~7.5;
preferably, the injection does not contain disodium edetate or calcium disodium edetate.

11. An injection
comprising:
a GLP-1-Fc-FGF21 fusion protein, a citrate buffer, mannitol, and optionally disodium edetate or calcium disodium edetate, as well as polysorbate 80 or polysorbate 20;
wherein the GLP-1-Fc-FGF21 fusion protein comprises an amino acid sequence as shown in any one of SEQ ID NO: 1~12, and the concentration of the GLP-1-Fc-FGF21 fusion protein in the injection is 25~100 mg/mL, preferably 25~95 mg/mL, 25~90 mg/mL, 25~85 mg/mL, 25~80 mg/mL, 25~75 mg/mL, 25~70 mg/mL, 25~65 mg/mL, 25~60 mg/mL, 25~55 mg/mL, or 25~50 mg/mL;
the concentration of the citrate buffer in the injection is 8~25 mM, preferably 10~20 mM;
the concentration of mannitol in the injection is 35~45 mg/mL;
the concentration of polysorbate 80 in the injection is 0.4~1.5 mg/mL or 0.4~1.3 mg/mL, or the concentration of polysorbate 20 in the injection is 0.1~0.5 mg/mL;
the concentration of the metal chelating agent in the injection is 0.05~0.5 mg/mL;
the pH of the injection is 6.9~7.5;
preferably, the injection does not contain disodium edetate or calcium disodium edetate.

12. An injection comprising:
a GLP-1-Fc-FGF21 fusion protein, a citrate buffer, mannitol, polysorbate 80, and optionally disodium edetate or calcium disodium edetate;
wherin the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL;
the concentration of the citrate buffer in the injection is 5~20 mM, preferably 8~20 mM, more preferably 10~15 mM;
the concentration of mannitol in the injection is 40~50 mg/mL;
the concentration of polysorbate 80 in the injection is 0.1~1.5 mg/mL;
the concentration of the metal chelating agent in the injection is 0.05~0.5 mg/mL;
the pH of the injection is 6.4~8.0;
preferably, the injection does not contain disodium edetate or calcium disodium edetate.

13. An injection comprising:
a GLP-1-Fc-FGF21 fusion protein, a citrate buffer, polysorbate 80, and sucrose or trehalose, and optionally disodium edetate or calcium disodium edetate;
wherein the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL;
the concentration of the citrate buffer in the injection is 5~20 mM, preferably 8~20 mM, more preferably 10~15 mM;
the concentration of sucrose in the injection is 85~95 mg/mL;
the concentration of trehalose in the injection is 55~65 mg/mL;
the concentration of polysorbate 80 in the injection is 0.1~1.5 mg/mL;
the pH of the injection is 6.4~8.0;
preferably, the injection does not contain disodium edetate or calcium disodium edetate.

14. An injection comprising:
a GLP-1-Fc-FGF21 fusion protein, a histidine buffer, mannitol, polysorbate 80, and optionally disodium edetate or calcium disodium edetate;
wherein the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL;
the concentration of the histidine buffer in the injection is 5~30 mM, preferably 10~20 mM;
the concentration of mannitol in the injection is 40~50 mg/mL;
the concentration of polysorbate 80 in the injection is 0.1~1.5 mg/mL;
the concentration of the metal chelating agent in the injection is 0.05~0.5 mg/mL;
the pH of the injection is 6.4~8.0;
preferably, the injection does not contain disodium edetate or calcium disodium edetate.

15. An injection comprising:
a GLP-1-Fc-FGF21 fusion protein, a histidine buffer, polysorbate 80, and sucrose or trehalose, and optionally disodium edetate or calcium disodium edetate;
the GLP-1-Fc-FGF21 fusion protein comprises an amino acid sequence as shown in any one of SEQ ID NO: 1~12, and the concentration of the GLP-1-Fc-FGF21 fusion protein in the injection is 3~25 mg/mL;
the concentration of the histidine buffer in the injection is 5~30 mM, preferably 10~20 mM;
the concentration of sucrose in the injection is 85~95 mg/mL;
the concentration of trehalose in the injection is 55~65 mg/mL;
the concentration of polysorbate 80 in the injection is 0.1~1.5 mg/mL;
the pH of the injection is 6.4~8.0;
preferably, the injection does not contain disodium edetate or calcium disodium edetate.

16. The injection according to any one of claims 8 to 15, further comprising a preservative.

17. The injection according to claim 16, wherein the preservative is at least one selected from phenol, m-cresol, benzyl alcohol, and phenoxyethanol;
optionally, the concentration of phenol in the composition or injection is 0~7 mg/mL, preferably 3~7 mg/mL;
optionally, the concentration of m-cresol in the composition or injection is 0~4 mg/mL, preferably 2~4 mg/mL.

18. Use of the composition according to any one of claims 1 to 7 or the injection according to any one of claims 8 to 17 in the preparation of a medicament for treating and/or preventing metabolic diseases;
optionally, the metabolic diseases include diabetes, hyperlipidemic obesity, and diseases associated with fatty liver;
optionally, the diseases associated with fatty liver include non-alcoholic fatty liver disease (NAFLD), non-alcoholic steatohepatitis (NASH), liver fibrosis, and cirrhosis.

19. A method of treating and/or preventing metabolic diseases, wherein the method comprising administering to a subject the composition according to any one of claims 1 to 7 or the injection according to any one of claims 8 to 17 in the preparation of a medicament for treating and/or preventing metabolic diseases;
optionally, the metabolic diseases include diabetes, hyperlipidemic obesity, and diseases associated with fatty liver;
optionally, the diseases associated with fatty liver include non-alcoholic fatty liver disease (NAFLD), non-alcoholic steatohepatitis (NASH), liver fibrosis, and cirrhosis.

20. The composition according to any one of claims 1 to 7 or the injection according to any one of claims 8 to 17 for use in treating and/or preventing metabolic diseases;
optionally, the metabolic diseases include diabetes, hyperlipidemic obesity, and diseases associated with fatty liver;
optionally, the diseases associated with fatty liver include non-alcoholic fatty liver disease (NAFLD), non-alcoholic steatohepatitis (NASH), liver fibrosis, and cirrhosis.

21. An injection device or container comprising:
the composition according to any one of claims 1 to 7 or the injection according to any one of claims 8 to 17;
optionally, the injection device or container comprises at least one selected from bottle, a vial, a cartridge, and a syringe;
preferably, the syringe comprises at least one selected from an auto-injector and a prefilled syringe.
